(19) Europäisches Patentamt
European Patent Office
Office européen des brevets

(11) Numéro de publication: 0 104 104
B1

(12) # FASCICULE DE BREVET EUROPÉEN

(45) Date de publication du fascicule du brevet: 24.09.86

(21) Numéro de dépôt: 83401696.6

(22) Date de dépôt: 23.08.83

(51) Int. Cl.⁴: **C 07 D 487/04**, C 07 D 239/46, C 07 D 239/70, C 07 D 239/95, A 61 K 31/505 // (C07D487/04, 239:00, 235:00)

(54) **Nouvelles imidazo/1,2-a/pyrimidines et leurs sels, leur procédé et leurs intermédiaires de préparation, leur application à titre de médicaments et les compositions les renfermant.**

(30) Priorité: 27.08.82 GB 8224606
09.02.83 GB 8303612

(43) Date de publication de la demande: 28.03.84 Bulletin 84/13

(45) Mention de la délivrance du brevet: 24.09.86 Bulletin 86/39

(84) Etats contractants désignés: AT BE CH DE FR IT LI LU NL SE

(56) Documents cité:
EP-A-0 061 380

IL FARMACO - ED. SC., vol. 35, fasc. 8, 1980, Pavia (IT) E. ABIGNENTE et al.: *"Research on heterocyclic compounds - IX - Heteroarylcarboxamides and acetamides: antiinflammatory and related pharmacological properties"*, pages 654-673

(73) Titulaire: **ROUSSEL- UCLAF, 35, boulevard des Invalides, F-75007 Paris (FR)**

(72) Inventeur: **Tully, Wilfred Roger, 5 Saint Peter's Road, Cirencester Gloucestershire (GB)**

(74) Mandataire: **Bourgouin, André, Département des Brevets ROUSSEL UCLAF B.P. no 9, F-93230 Romainville (FR)**

EP 0 104 104 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

La présente invention concerne de nouvelles imidazo /1,2-a/ pyrimidines et leurs sels, ainsi que le procédé et les intermédiaires de préparation, l'application à titre de médicaments de ces nouveaux produits et les compositions les renfermant.

L'invention a pour objet de nouvelles imidazo /1,2-a/ pyrimidines répondant à la formule (I):

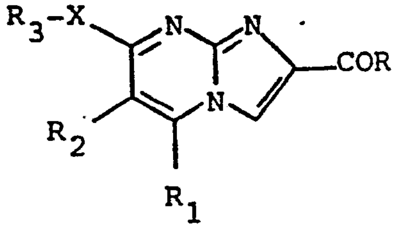

(I)

dans laquelle R représente un radical alcoyle linéaire renfermant de 1 à 8 atomes de carbone, un radical alcényle linéaire renfermant de 2 à 8 atomes de carbone, un radical cycloalcoyle renfermant de 3 à 7 atomes de carhone, un radical (cycloalcoyl) alcoyle renfermant de 4 à 12 atomes de carbone, un radical aryle renfermant de 6 à 12 atomes de carbone ou un radical thiényle ou pyridyle, chacun des radicaux ci-dessus pouvant être substitué par un ou plusieurs atomes d'halogènes, un ou plusieurs radicaux alcoyles, alcoylthio ou alcoxy renfermant de 1 à 5 atomes de carbone, un ou plusieurs radicaux amino, alcoylamino ou dialcoylamino, dans lesquels les radicaux alcoylesrenferment de 1 à 5 atomes de carbone, ou par un ou plusieurs radicaux trifluorométhyles,

$R_1$ et $R_2$, identiques ou différents, représentent un atome d'hydrogène, un radical alcoyle linéaire renfermant de 1 à 8 atomes de carbone, un radical alcényle linéaire renfermant de 2 à 5 atomes de carbone, un radical cycloalcoyle renfermant de 3 à 7 atomes de carbone, un radical (cycloalcoyl) alcoyle renfermant de 4 à 12 atomes de carbone, un radical benzyle ou un radical phénéthyle ou $R_1$ et $R_2$ forment ensemble un radical alcoylène linéaire renfermant de 3 à 5 atomes de carbone, chacun des radicaux ci-dessus pouvant être substitué par un ou plusieurs atomes d'halogènes, un ou plusieurs radicaux alcoyles alcoxy ou alcoylthio renfermant de 1 à 5 atomes de carbone, par un ou plusieurs radicaux amino, alcoylamino, ou dialcoylamino, dans lesquels les radicaux alcoyles renfermant de 1 à 5 atomes de carbone, ou par un ou plusieurs radicaux trifluorométhyle;

X représente un atome d'oxygène ou de soufre et $R_3$ représente un radical alcoyle renfermant de 1 à 5 atomes de carbone, ainsi que leurs sels d'addition avec les acides minéraux ou organiques.

Dans la formule générale (1) et dans ce qui suit:

- par un radical alcoyle renfermant de 1 à 8 atomes de carbone on entend, de préférence, ceux renfermant de 1 à 5 atomes de carbone, plus particulièrement un radical méthyle, éthyle, propyle, butyle ou pentyle ou, lorsque le radical alcoyle est substitué par un radical alcoyle, un radical isopropyle, isobutyle ou terbutyle;
- par un radical alcényle on entend, de préférence, un radical vinyle, allyle, but-3-ényle ou, lorsque le radical alcényle est substitué par un radical alcoyle, un radical isopropényle;
- par un radical cycloalcoyle on entend de préférence un radical cyclopropyle, cyclobutyle, cyclopentyle, cyclohexyle ou cycloheptyle;
- par un radical (cycloalcoyl) alcoyle, on entend de préférence l'un des radicaux cycloalcoyle ci-dessus, substitué par l'un des radicaux alcoyle ci-dessus;
- par un radical aryle, on entend de préférence un radical phényle ou naphthyle.

Les sels d'addition avec les acides minéraux ou organiques peuvent être, par exemple, les sels formes avec les acides chlorhydrique bromhydrique, iodhydrique, nitrique, sulfurique, phosphorique, propionique, formique, benzoïque, maléique, fumarique, succinique, tartrique, citrique, oxalique, glyoxylique, aspartique, alcanesulfoniques tels que l'acide méthanesulfonique et arylsulfoniques, tels que l'acide benzènesulfonique.

Parmi les produits, objet de l'invention, on peut citer notamment les dérivés répondant à la formule (I) ci-dessus, ainsi que leurs sels, caractérisés en ce que dans ladite formule (I), R représente un radical phényle, $R_1$, $R_2$, $R_3$ et X ont la signification déjà indiquée.

Parmi ces derniers, on peut citer les dérivés répondant à la formule (I) ci-dessus, ainsi que leurs sels, caractérisés en ce que dans ladite formule (I), $R_1$ et $R_2$, identiques ou differents, representent un atome d'hydrogène, un radical méthyle, éthyle, propyle, isopropyle, hexyle, allyle, benzyle ou $R_1$ et $R_2$ forment ensemble un radical 1, 3-propylène, 1, 4-butylène, 1, 5-pentylène ou un radical

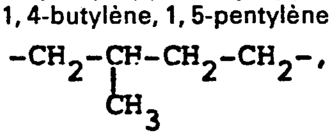

, $R_3$ représente un radical méthyle ou éthyle, R et X ayant la signification déjà indiquée.

Parmi les produits, objet de l'invention, on retient tout particulièrement ceux dont les noms suivent:

- la /6-éthyl 5-méthyl 7- (méthylthio) imidazo /1, 2-a/ pyrimidin-2-yl/ phénylméthanone;
- la (5-méthoxy 6, 7, 8, 9-tétrahydroimidazo /1, 2-a/ quinazolin-2-yl) phénylméthanone;

- la /5- (méthylthio) cyclopenta /e/ imidazo /1, 2-a/ pyrimidin-2-yl/ phénylméthanone;
- la /7-méthoxy 5-méthyl 6- (1-méthyléthyl) imidazo /1, 2-a/ pyri-midin-2-yl/ phénylméthanone et
- la (6-éthyl 7-méthoxy 5-méthyl imidazo /1, 2-a/ pyrimidin-2-yl) phénylméthanone ainsi que leurs sels.

L'invention a également pour objet un procédé de préparation des nouvelles imidazo /1, 2-a/ pyrimidines telles que définies par la formule (I) ci-dessus, ainsi que de leurs sels, caractérisé en ce que l'on fait réagir un produit de formule (II):

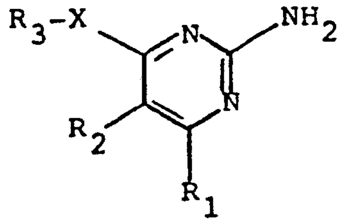

(II)

dans laquelle RI, $R_2$, $R_3$ et X ont la signification déjà indiquée avec un produit de formule (III):

$Y-CH_2-CO-CO-R$ (III)

dans laquelle R a la signification déjà indiquée et Y est un groupement pouvant former, par élimination, un anion Yθ, pour obtenir un produit de formule (IV):

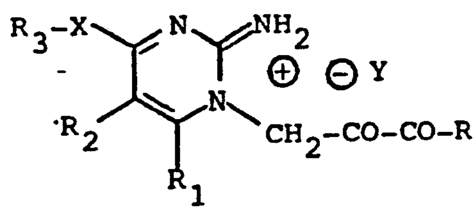

(IV)

dans laquelle R, $R_1$, $R_2$, $R_3$, X et Y ont la signification déjà indiquée, puis cyclise ledit produit de formule (IV) obtenu pour obtenir un produit de formule (I) que l'on peut salifier le cas échéant.

Dans des conditions préférentielles de mise en oeuvre de l'invention, le procédé de préparation ci-dessus décrit est caractérisé en ce que:

a) la réaction du produit de formule (II) avec le produit de formule (III) est effectuée au sein d'un solvant organique tel que l'éther éthylique, le tétrahydrofuranne ou le diméthoxyéthane;

b) la cyclisation du produit de formule (IV) avec ou sans isolement intermédiaire est effectuée au reflux du mélange réactionnel au sein d'un solvant tel que le méthanol ou par agitation à température ambiante, au sein d'un solvant tel que le méthanol, en présence d'une base telle que le carbonate de potassium.

En général, la cyclisation ci-dessus conduit à la formation d'un sel d'acide HY. La base libre peut être facilement obtenue par action d'une base telle qu'un hydroxyde ou un carbonate alcalin, par exemple de potassium. La base libre obtenue peut à son tour être salifiée si désiré. Certains produits de formule (II) sont décrits dans la littérature. Lorsqu'ils ne sont pas connus, ces produits peuvent notamment être préparés selon les procédés décrits dans J.A. C. S., 1959, 81, 3108; dans Rec. Trav. Chim., 1942, 61, 291 ou dans 5 Tetrahedron, 1976, 32, 1779.

De même, certains produits de formule (III) sont décrits dans la littérature. Lorsqu'ils ne sont pas connus, ces produits peuvent notamment être préparés selon les procédés décrits dans Helv. Chim. Acta., 1846, 29, 1247 ou dans le brevet américain n° 2 821 555.

L'invention a également pour objet une variante du procédé de préparation des produits de formule (I) et de leurs sels, caractérisé en ce que l'on fait réagir un produit de formule (II)

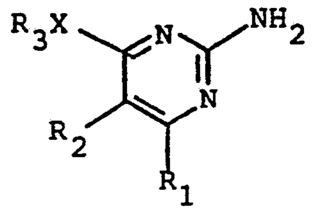

(II)

dans laquelle $R_1$, $R_2$, $R_3$ et X ont la signification déjà indiquée, avec un produit de formule
$A\text{-}CH_2\text{-}CO\text{-}CO_2Q$
dans laquelle A représente un groupement nucléophile et Q représente un groupement estérifiant, cyclise le produit obtenu de formule

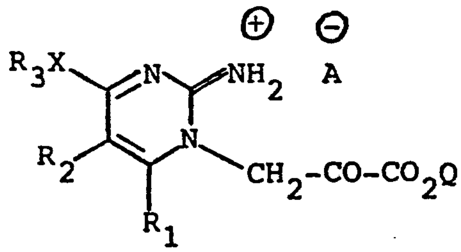

dans laquelle $R_1$, $R_2$, $R_3$, Q et X ont la signification déjà indiquée et $A^{\theta}$ représente un anion dérivé de A, pour obtenir le produit de formule (IX):

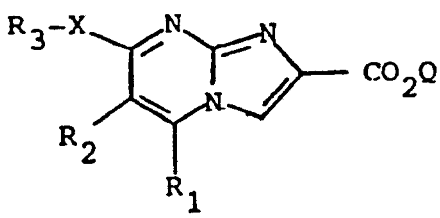

(IX)

dans laquelle $R_1$, $R_2$, $R_3$, X et Q ont la signification déjà indiquée, réduit le produit de formule (IX) pour obtenir le produit de formule (VIII):

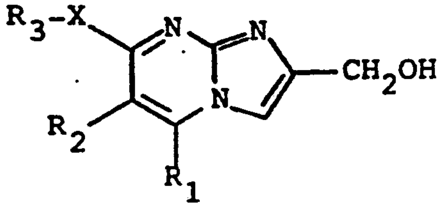

(VIII)

dans laquelle $R_1$, $R_2$, $R_3$ et X ont la signification déjà indiquée, oxyde le produit de formule (VIII) pour obtenir le produit de formule (VI):

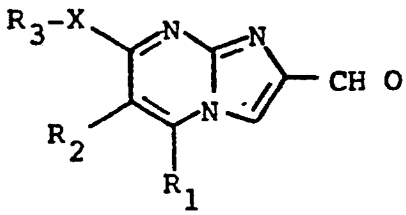

(VI)

dans laquelle $R_1$, $R_2$, $R_3$ et X ont la significations déjà indiquée, fait réagir le produit de formule (VI) avec un dérivé magnésien d'un produit de formule (VII):

R-K (VII)

dans laquelle R a la signification déjà indiquée et K représente un atome de lithium ou un reste -MgHal, dans lequel Hal représente un atome de chlore, de brome ou d'iode, pour obtenir un produit de formule (V):

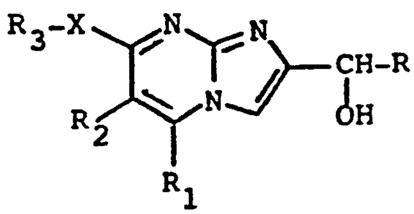

. (V)

dans laquelle R, $R_1$, $R_2$, $R_3$ et X ont la signification déjà indiquée, puis oxyde le produit de formule (V) pour obtenir le produit de formule (I) attendu que l'on peut salifier, le cas échéant.

Dans des conditions préférentielles de mise en oeuvre de l'invention, le procédé de préparation ci-dessus décrit est caractérisé en ce que:

a) La cyclisation menant au produit de formule (IX) est effectuée dans les mêmes conditions que celle du produit de formule (IV).

b) Q est un radical alcoyle ou aralcoyle et plus particulièrement un radical alcoyle renfermant de 1 à 3 atomes de carbone tel qu'un radical éthyle.

c) La réduction du produit de formule (IX) est effectuée par l'hydrure de lithium-aluminium ou encore par le borohydrure de sodium en présence de chlorure d'aluminium ou le borohydrure de lithium.

d) L'oxydation du produit de formule (VIII) est effectuée de manière conventionnelle, par exemple à l'aide de dioxyde manganèse. On peut encore utiliser l'un des agents d'oxydation utilisés pour oxyder le produit de formule (V) et indiqués ci-après.

e) La réaction du produit de formule (VI) avec le produit de formule (VII) est effectuée dans des conditions anhydres dans un solvant organique tel que le tétrahydrofuranne.

f) L'oxydation du produit de formule (V) est effectuée avec le dioxyde de manganèse, mais on peut également utiliser l'acide nitrique, le chlorure ferrique ou l'oxyde de chrome en présence de pyridine ou encore la méthode d'Oppenauer ou enfin une déshydrogénation en présence d'un catalyseur à base de cuivre.

Certains produits de formule (IX) sont décrits dans Farmaco. Ed. Sci. 1977, 32 (10), 735 et 1980, 35 (8), 654.

Les produits de formule (I) présentent un caractère basique. On peut avantageusement préparer les sels d'addition des produits de formule (I) en faisant réagir, en proportions sensiblement stoéchiométriques, un acide minéral ou organique avec lesdits produits de formule (I). Les sels peuvent être préparés sans isoler les bases correspondantes.

Les produits, objet de la présente invention, possèdent de très intéressantes propriétés pharmacologiques; ils sont doués notamment de remarquables propriétés anxiolytiques.

Ces propriétés sont illustrées plus loin dans la partie expérimentale.

Ces propriétés justifient l'utilisation des nouvelles imidazo /1,2-a/ pyrimidines de formule (I), ainsi que de leurs sels, pharmaceutiquement acceptables, à titre de médicaments.

La présente invention a ainsi également pour objet l'application à titre de médicaments des nouvelles imidazo /1,-2-a/ pyrimidines telles que définies par la formule générale (I) ainsi que de leurs sels d ' addition avec les acides pharmaceutiquement acceptables.

Parmi les médicaments, objet de l'invention, on retient notamment les médicaments caractérisés en ce qu'ils sont constitués par les nouvelles imidazo /1, 2-a/ pyrimidines répondant à la formule (I) dans laquelle R représente un radical phényle, $R_1$, $R_2$, $R_3$ et X ont la signification déjà indiquée, ainsi que par leurs sels d'addition avec les acides pharmaceutiquement acceptables.

Parmi les médicaments, objet de l'invention, on retient tout particulièrement ceux répondant à la formule (I), caractérisés en ce que dans ladite formule (I), $R_1$ et $R_2$, identiques ou différents, représentent un atome

d'hydrogène, un radical méthyle, éthyle, propyle, isopropyle, hexyle; allyle, benzyle ou $R_1$ et $R_2$ forment ensemble un radical 1, 3-propylène, I, 4-butylène, 1, 5-pentylène ou un radical $-CH_2-$

CH
|
$CH_3$

$-CH_2-CH_2-$, $R_3$ représente un radical méthyle ou éthyle, R et X ayant la signification déjà indiquée ainsi que leurs sels d'addition avec les acides pharmaceutiquement acceptables.

Parmi les médicaments préférés de l'invention, on retient tout particulièrement:

- la /6-éthyl 5-méthyl 7- (méthylthio) imidazo /1, 2-a/ pyrimidin-2-yl/ phénylméthanone;
- la (5-méthoxy 6, 7, 8, 9-tétrahydroimidazo /1, 2-a/ quinazolin-2-yl) phénylméthanone;
- la / (5-méthylthio) cyclopenta /e/ imidazo /I, 2-a/ pyrimidin-2-yl/ phénylméthanone;
- la /7-méthoxy 5-méthyl 6- (1-méthyléthyl) imidazo /I, 2-a/ pyri-midin-2-yl/ phénylméthanone et
- la (6-éthyl 7-méthoxy 5-méthyl imidazo /1, 2-a/ pyrimidin-2-yl) phénylméthanone ainsi que leurs sels pharmaceutiquement acceptables.

Ces médicaments trouvent, par exemple, leur emploi dans le traitement des états anxieux, des anxiétés chroniques avec agitation, irritabilité, agressivité, des anxiétés avec insomnies et tensions musculaires, des angoisses.

La dose usuelle, variable selon le produit utilisé, le sujet traité et l'affection en cause peut être par exemple, de 0,1 mg à 200 mg par jour, par voie orale.

L'invention a également pour objet les compositions pharmaceutiques qui renferment au moins un dérivé précité ou l'un de ses sels d'addition avec les acides phamaceutiquement acceptablesà titre de principe actif.

A titre de médicaments, les dérivés répondant à la formule (I) et leurs sels d 'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple les comprimés, simples ou dragéifiés, les gélules, les capsules, les granulés, les suppositoires, les préparations injectables; elles sont préparées selon les méthodes usuelles. Le ou les principes actifs peuvent y être incorporés à des excipients habituellement employés dans ces compositions pharmaceutiques, tels que le talc, la gomme arabique, le lactose, l 'amidon, le stéarate de magnésium, le beurre de cacao, les véhicules aqueux ou non les corps gras d ' origine animale ou végétale les dérivés paraffiniques, les glycols, les divers agents mouillants, dispersants ou émulsifiants, les conservateurs.

L'invention a enfin pour objet, à titre de produits nouveaux et notamment d'intermédiaires nécessaires à la préparation des produits de formule (I), les produits de formule (IV), ainsi que les produits de formules (V), (VI) et (VIII) que l 'on peut représenter par la formule

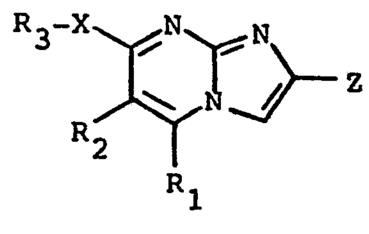

dans laquelle $R_1$, $R_2$, $R_3$ et X sont définis comme précédemment et Z représente un radical -CHO ou

-CH
|
OH

-R' dans lequel R' représente un atome d'hydrogène ou un radical R tel que défini précédemment.

Les exemple suivants illustrent l'invention sans toutefois la limiter.

**Exemple 1: 5-méthoxy 6,7,8,9-tétrahydroimidazo/1,2-a/quinazolin-2-yl)phénylméthanone**

On agite à température ambiante pendant une nuit une solution de 20 g de 4-méthoxy 5,6,7,8-tétrahydro-2-quinazolinamine et de 28,6 g de 3-bromo 1-phénylpropane-1,2-dione dans 400 $cm^3$ d'éther éthylique. On filtre le bromure de 4-méthoxy 1-(3-phényl 2,3-dioxopropyl) 5, 6, 7, 8-tétrahydro-2-quinazolinium qui a précipité, le lave à l'éther, le met en suspension dans le méthanol et porte au reflux pendant deux heures.

On évapore à sec la solution obtenue sous pression réduite, reprend le résidu dans un mélange de

chloroforme et de solution aqueuse de carbonate de potassium. On lave la phase organique, la sèche et l'évapore à sec sous pression réduite.

On triture le résidu dans le méthanol, filtre les cristaux formés et obtient 18 g de produit attendu. F = 234°.

**Exemples 2 à 48**

En utilisant une méthode analogue à celle décrite à l'exemple 1 mais en partant des composés correspondants de formule II dans laquelle $R_1$, $R_2$, $R_3$ et X ont les significations indiquées dans le tableau I ci-après, on a préparé les produits des exemples 2 à 48 (voir tableau I ci-après). Les analyses spectrométriques, les rendements et les points de fusion des composés sont aussi donnés dans le tableau I. La microanalyse est donnée dans le tableau II.

**Exemple 2:**

5-éthoxy 6, 7, 8, 9-tétrahydroimidazo/1,2-a/quinozlin-2-yl) phénylméthanone.

**Exemple 3**

7-méthoxy 5-méthylimidazo/1,2-a/pyrimidin-2-yl)phényl-méthanone.

**Exemple 4**

5-méthoxycyclopenta/e/imidazo/1,2-a/pyrimidin-2-yl)phényl-méthanone.

**Exemple 5**

5-éthoxycyclopenta/e/imidazo/1,2-a/pyrimidin-2-yl)phényl-méthanone.

**Exemple 6**

5-(méthylthio)cyclopenta/e/imidazo/1,2-a/pyrimidin-2-yl) phénylméthanone.

**Exemple 7**

7-méthoxy 5,6- diméthylmidaso/1,2-a/pyrimidin-2-yl) phénylméthanone.

**Exemple 8**

5-méthoxycyclohepta/e/imidaso/1,2-a/pyrimidin-2-yl) phénylméthanone.

**Exemple 9**

6-benzyl 7-méthoxy 5-méthylimidaso/1,2-a/pyrimidin-2-yl) phénylméthanone.

**Exemple 10**

5-éthoxycyclohepta/e/imidaso/1,2-a/pyrimidin-2-yl) phénylméthanone.

**Exemple 11**

7-méthoxy 5-méthyl 6-(1-m51thyléthyl)imidaso/1,2-a/pyrimidin-2-yl)phénylméthanone.

**Exemple 12**

5-(méthylthio) 6,7,8,9-tétrahydroimidaso/1,2-a/quinazolin-2-yl)phénylméthanone.

**Exemple 13**

5-(éthylthio)cyclopenta/e/imidaso/1,2-a/pyrimidin-2-yl) phénylméthanone.

**Exemple 14**

7-méthoxy 5-propylimidaso/1,2-a/pyrimidin-2-yl)phényl-méthanone.

**Exemple 15**

5-éthyl 7-méthoxyimidaso/1,2-a/pyrimidin-2-yl)phénylmétha-none.

**Exemple 16**

6-éthyl 7-méthoxy 5-méthylimidaso/1,2-a/pyrimidin-2-yl) phénylméthanone.

**Exemple 17**

5-méthoxy 8-méthyl 6,7,8,9-tétrahydroimidaso/1,2-a/ quinazolin-2-yl)phénylméthanone.

**Exemple 18**

8-méthyl 5-(méthylthio)-6,7,8,9-tétrahydroimidaso/1,2-a/ quinazolin-2-yl)phénylméthanone.

**Exemple 19**

6-n-hexyl 7-méthoxy 5-méthylimidaso/1,2-a/pyrimidin-2-yl) phénylméthanone.

**Exemple 20**

6-allyl 7-méthoxy 5-méthylimidazo /1,2-a/pyrimidon-2-yl) phénylméthanone.

**Exemple 21**

5-méthyl 7-(méthylthio)imidazo/1,2-a/pyrimidin-2-yl) phénylméthanone.

**Exemple 22**

7-(méthylthio) 5-propylimidazo/1,2-2/pyrimidin-2-yl) phénylméthanone.

**Exemple 23**

(6-cyclopropylméthyl)7-méthoxy 5-méthylimidazo/1,2-a/ pyrimidin-2-yl)phénylméthanone.

**Exemple 24**

(6-cyclohexylméthyl)7-méthoxy 5-méthylimidazo/1,2-a/pyrimidin-2-yl)phénylméthanone.

**Exemple 25**

7-éthoxy 6-éthyl 5-méthylimidazo/1,2-a/pyrimidin-2-yl)phénylméthanone.

**Exemple 26**

7-méthoxy 6-méthyl 5-propylimidazo/1,2-a/pyrimidin-2-yl)phénylméthanone.

**Exemple 27**

6-éthyl 7-méthoxy 5-n-propylimidazo/1,2-a/pyrimidin-2-yl)phénylméthanone.

**Exemple 28**

7-méthoxyimidazo/1,2-a/pyrimidin-2-yl)phénylméthanone.

**Exemple 29**

5-éthyl 7-méthoxy 6-méthylimidazo/1,2-a/pyrimidin-2-yl)phénylméthanone.

**Exemple 30**

6-n-butyl 7-méthoxy 5-métylimidazo/1,2-a/pyrimidin-2-yl)phénylméthanone.

**Exemple 31**

5,6-diéthyl 7-méthoxyimidazo/1,2-a/pyrimidin-2-yl)phénylméthanone.

**Exemple 32**

7-méthoxy 5-méthyl 6-n-propylimidazo/1,2-a/pyrimidin-2-yl)méthanone.

**Exemple 33**

7-méthoxy 5,6-di-n-propylimidazo/1,2-a/pyrimidin-2-yl)phénylméthanone.

**Exemple 34**

6-n-butyl 7-méthoxy 5-n-propylimidazo/1,2-a/pyrimidin-2-yl)phénylméthanone.

**Exemple 35**

5-éthyl 7-méthoxy 6-n-propylimidazo/1,2-a/pyrimidin-2-yl)phénylméthanone.

**Exemple 36**

6-n-butyl 5-éthyl 7-méthoxy imidazo/1,2-a/pyrimidin-2-yl)phénylméthanone.

**Exemple 37**

6-allyl 7-méthoxy 5-n-propylimidazo/1,2-a/pyrimidin-2-yl)phénylméthanone.

**Exemple 38**

6-éthyl 7-méthoxyimidaso/1,2-a/pyrimidin-2-yl)phényl)méthanone.

**Exemple 39**

5-n-butyl 7-méthoxy 6-méthylimidaso/1,2-a/pyrimidin-2-yl)phénylméthanone.

**Exemple 40**

7-méthoxy 6-n-propylimidaso/1,2-a/pyrimidin-2-yl)phénylméthanone.

**Exemple 41**

6-n-butyl 7-méthoxyimidaso/1,2-a/pyrimidin-2-yl)phénylméthanone.

**Exemple 42**

7-méthoxy 6-méthylimidaso/1,2-a/pyrimidin-2-yl)phénylméthanone.

**Exemple 43**

5-n-butyl 7-méthoxyimidaso/1,2-a/pyrimidin-2-yl)phénylméthanone.

**Exemple 44**

6-allyl 5-éthyl 7-méthoxyimidaso/1,2-a/pyrimidin-2-yl)phénylméthanone.

**Exemple 45**

5-n-butyl 6-éthyl 7-méthoxyimidaso/1,2-a/pyrimidin-2-yl)phénylméthanone.

**Exemple 46**

5-n-butyl 7-méthoxy 6-n-propylimidaso/1,2-a/pyrimidin-2-yl)phénylméthanone.

**Exemple 47**

7-méthoxy 5-méthyl 6-n-pentylimidaso/1,2-a/pyrimidin-2-yl)phénylméthanone.

**Exemple 48**

6-isobutyl 7-méthoxy 5-méthylimidaso/1,2-a/pyrimidin-2-yl)phénylméthanone.

Tableau I

| Ex No | R | $R_1$ | $R_2$ | $R_3X$ | Rdt % | IR $cm^{-1}$ (KBr) | F °C |
|---|---|---|---|---|---|---|---|
| 1 | Ph | $-(CH_2)_4-$ | | $CH_3O$ | 52 | 3120,1640 | 234 |
| 2 | Ph | $-(CH_2)_4-$ | | $C_2H_5O$ | 48 | 3120,1640 | 250-2 |
| 3 | Ph | $CH_3$ | H | $CH_3O$ | 43 | 3155,1650 | 165-6 |
| 4 | Ph | $-(CH_2)_3-$ | | $CH_3O$ | 35 | 3100,1655 | 210-1 |
| 5 | Ph | $-(CH_2)_3$ | | $C_2H_5O$ | 47 | 3105,1640 | 227-9 |
| 6 | Ph | $-(CH_2)_3-$ | | $CH_3S$ | 44 | 3110,1640 | 208-9 |
| 7 | Ph | $CH_3$ | $CH_3$ | $CH_3O$ | 76 | 3140,1650 | 198-205 |
| 8 | Ph | $-(CH_2)_5-$ | | $CH_3O$ | 44 | 3120,1630 | 196-8 |
| 9 | Ph | $CH_3$ | $CH_2Ph$ | $CH_3O$ | 55 | 3110,1640 | 199-202 |
| 10 | Ph | $-(CH_2)_5-$ | | $C_2H_5O$ | 39 | 3130,1640 | 193-4 |
| 11 | Ph | $CH_3$ | $CH(CH_3)_2$ | $CH_3O$ | 48 | 3120,1640 | 148-50 |
| 12 | Ph | $-(CH_2)_4-$ | | $CH_3S$ | 35 | 3180,1639 | 225 |
| 13 | Ph | $-(CH_2)_3$ | | $C_2H_5S$ | 63 | 3100,1630 | 211-2 |
| 14 | Ph | $C_3H_7$ | H | $CH_3O$ | 37 | 2950,1640 | 152-4 |
| 15 | Ph | $C_2H_5$ | H | $CH_3O$ | 49 | 3110,1640 | 179-82 |
| 16 | Ph | $CH_3$ | $C_2H_5$ | $CH_3O$ | 68 | 3120,1640 | 148-50 |
| 17 | Ph | $-CH_2CH(CH_3)CH_2CH_2-$ | | $CH_3O$ | 43 | 3110,1640 | 200-1 |
| 18 | Ph | $-CH_2CH(CH_3)CH_2CH_2-$ | | $CH_3S$ | 52 | 3110,1630 | 215-7 |
| 19 | Ph | $CH_3$ | n-hexyl | $CH_3O$ | 42 | 3130,1630 | 122-6 |
| 20 | Ph | $CH_3$ | $CH_2=CHCH_2$ | $CH_3O$ | 68 | 3120,1640 | 128-31 |
| 21 | Ph | $CH_3$ | H | $CH_3S$ | 58 | 3100,1640 | 170-2 |
| 22 | Ph | $C_3H_7$ | H | $CH_3S$ | 72 | 3160,1650 | 162-3 |

**Tableau I (suite)**

| Ex. No. | R | $R_1$ | $R_2$ | $R_3X$ | Rdt % | $IRcm^{-1}$ (KBr) | F °C |
|---|---|---|---|---|---|---|---|
| 23 | Ph | $CH_3$ | 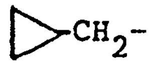 | $CH_3O$ | 32 | 3120,1640 | 136-7 |
| 24 | Ph | $CH_3$ | 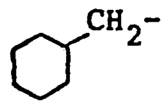 | $CH_3O$ | 55 | 3130,1645 | 169-70 |
| 25 | Ph | $CH_3$ | $C_2H_5$ | $C_2H_5O$ | 23 | 3120,1640 | 154-9 |
| 26 | Ph | $C_3H_7$ | $CH_3$ | $CH_3O$ | 40 | 3120,1640 | 140-1 |
| 27 | Ph | $C_3H_7$ | $C_2H_5$ | $CH_3O$ | 36 | 3120,1635 | 122-3 |
| 28 | Ph | H | H | $CH_3O$ | 84 | 3120,1640 | 208-9 |
| 29 | Ph | $C_2H_5$ | $CH_3$ | $CH_3O$ | 31 | 3120,1641 | 174 |
| 30 | Ph | $CH_3$ | $C_4H_9$ | $CH_3O$ | 26 | 3120,1640 | 141-5 |
| 31 | Ph | $C_2H_5$ | $C_2H_5$ | $CH_3O$ | 23 | 3155,1642 | 141-2 |
| 32 | Ph | $CH_3$ | $C_3H_7$ | $CH_3O$ | 54 | 3120,1640 | 144-5 |
| 33 | Ph | $C_3H_7$ | $C_3H_7$ | $CH_3O$ | 31 | 3130,1640 | 123-4 |
| 34 | Ph | $C_3H_7$ | $C_4H_9$ | $CH_3O$ | 30 | 3120,1635 | 113-4 |
| 35 | Ph | $C_2H_5$ | $C_3H_7$ | $CH_3O$ | 26 | 3120,1635 | 151-2 |
| 36 | Ph | $C_2H_5$ | $C_4H_9$ | $CH_3O$ | 20 | 3130,1635 | 158-9 |
| 37 | Ph | $C_3H_7$ | allyl | $CH_3O$ | 40 | 3140,1640 | 122-3 |
| 38 | Ph | H | $C_2H_5$ | $CH_3O$ | 52 | 3140,1650 | 126-9 |

Tableau I (suite)

| Ex. No. | R | $R_1$ | $R_2$ | $R_3X$ | Rdt % | IRcm$^{-1}$ (KBr) | F °C |
|---|---|---|---|---|---|---|---|
| 39 | Ph | $C_4H_9$ | $CH_3$ | $CH_3O$ | 14 | 3120,1640 | 122-4 |
| 40 | Ph | H | $C_3H_7$ | $CH_3O$ | 89 | 3160,1625 | 137-9 |
| 41 | Ph | H | $C_4H_9$ | $CH_3O$ | 48 | 3150,1660 | 149-50 |
| 42 | Ph | H | $CH_3$ | $CH_3O$ | 32 | 3040,1660 | 179-80 |
| 43 | Ph | $C_4H_9$ | H | $CH_3O$ | 39 | 3140,1650 | 170-1 |
| 44 | Ph | $C_2H_5$ | allyl | $CH_3O$ | - | 3130,1640 | 130-1 |
| 45 | Ph | $C_4H_9$ | $C_2H_5$ | $CH_3O$ | 17 | 3120,1640 | 124-5 |
| 46 | Ph | $C_4H_9$ | $C_3H_7$ | $CH_3O$ | 19 | 3140,1640 | 118-9 |
| 47 | Ph | $CH_3$ | $C_5H_{11}$ | $CH_3O$ | - | 3130,1640 | 150 |
| 48 | Ph | $CH_3$ | iso-$C_4H_9$ | $CH_3O$ | 17 | 3130,1640 | 160 |

**Tableau II**

| Ex No | Formule | pds mol. | Calculé/Trouvé C | H | N | X |
|---|---|---|---|---|---|---|
| 1 | $C_{18}H_{17}N_3O_2(\frac{1}{2}H_2O)$ | 307.4 | 68.33<br>68.5 | 5.73<br>5.5 | 13.28<br>13.5 | |
| 2 | $C_{19}H_{19}N_3O_2(\frac{1}{2}H_2O)$ | 321.4 | 69.07<br>69.3 | 6.10<br>5.9 | 12.72<br>12.8 | |
| 3 | $C_{15}H_{13}N_3O_2$ | 267.3 | 67.41<br>67.5 | 4.90<br>4.95 | 15.72<br>15.8 | |
| 4 | $C_{17}H_{15}N_3O_2$ | 293.3 | 69.61<br>69.8 | 5.15<br>5.2 | 14.33<br>14.5 | |
| 5 | $C_{18}H_{17}N_3O_2$ | 307.4 | 70.34<br>70.5 | 5.58<br>5.6 | 13.67<br>13.8 | |
| 6 | $C_{17}H_{15}N_3OS$ | 309.4 | 66.00<br>65.95 | 4.89<br>5.0 | 13.58<br>13.6 | 10.36(S)<br>10.4 |
| 7 | $C_{16}H_{15}N_3O_2$ | 281.3 | 68.31<br>67.9 | 5.37<br>5.4 | 14.94<br>14.9 | |
| 8 | $C_{19}H_{19}N_3O_2$ | 321.4 | 71.01<br>70.9 | 5.96<br>6.0 | 13.07<br>13.1 | |
| 9 | $C_{22}H_{19}N_3O_2$ | 357.4 | 73.93<br>74.0 | 5.36<br>5.4 | 11.76<br>11.8 | |
| 10 | $C_{20}H_{21}N_3O_2$ | 335.4 | 71.62<br>71.65 | 6.31<br>6.4 | 12.53<br>12.5 | |
| 11 | $C_{18}H_{19}N_3O_2$ | 309.4 | 69.88<br>69.5 | 6.19<br>6.3 | 13.58<br>13.5 | |
| 12 | $C_{18}H_{17}N_3OS(\frac{1}{4}H_2O)$ | 323.4 | 65.92<br>65.8 | 5.38<br>5.3 | 12.81<br>13.0 | 9.78(S)<br>9.85 |
| 13 | $C_{18}H_{17}N_3OS$ | 323.4 | 66.85<br>66.8 | 5.30<br>5.3 | 12.99<br>13.0 | 9.91 (S)<br>10.0 |
| 14 | $C_{17}H_{17}N_3O_2$ | 295.3 | 69.14<br>69.1 | 5.80<br>5.8 | 14.23<br>14.3 | |
| 15 | $C_{16}H_{15}N_3O_2$ | 281.3 | 68.31<br>68.3 | 5.37<br>5.4 | 14.94<br>15.0 | |
| 16 | $C_{17}H_{17}N_3O_2$ | 295.3 | 69.14<br>69.2 | 5.80<br>5.8 | 14.23<br>14.2 | |
| 17 | $C_{19}H_{19}N_3O_2$ | 321.4 | 71.01<br>71.1 | 5.96<br>6.0 | 13.07<br>13.0 | |
| 18 | $C_{19}H_{19}N_3OS$ | 337.4 | 67.63<br>67.7 | 5.68<br>5.7 | 12.45<br>12.5 | 9.49(S)<br>9.4 |
| 19 | $C_{21}H_{25}N_3O_2$ | 351.2 | 71.77<br>71.8 | 7.17<br>7.1 | 11.96<br>11.9 | |
| 20 | $C_{18}H_{17}N_3O_2$ | 307.4 | 70.34<br>70.2 | 5.58<br>5.6 | 13.67<br>13.6 | |
| 21 | $C_{15}H_{13}N_3OS$ | 283.4 | 63.61<br>63.5 | 4.62<br>4.7 | 14.83<br>14.8 | 11.32(S)<br>11.4 |
| 22 | $C_{16}H_{17}N_3OS$ | 311.4 | 65.57<br>65.3 | 5.50<br>5.5 | 13.49<br>13.55 | 10.30(S)<br>10.4 |

Tableau II (suite)

| Ex No. | Formule | Pds mol. | Calculé/Trouvé | | | |
|---|---|---|---|---|---|---|
| | | | C | H | N | X |
| 23 | $C_{19}H_{19}N_3O_2$ | 321.4 | 71.01<br>71.0 | 5.96<br>6.0 | 13.07<br>13.1 | |
| 24 | $C_{22}H_{25}N_3O_2$ | 363.5 | 72.70<br>72.7 | 6.93<br>7.0 | 11.56<br>11.55 | |
| 25 | $C_{18}H_{19}N_3O_2$ | 309.4 | 69.88<br>69.7 | 6.19<br>6.2 | 13.58<br>13.5 | |
| 26 | $C_{18}H_{19}N_3O_2$ | 309.4 | 69.88<br>69.7 | 6.19<br>6.2 | 13.58<br>13.5 | |
| 27 | $C_{19}H_{21}N_3O_2$ | 323.4 | 70.57<br>70.6 | 6.55<br>6.5 | 12.99<br>13.0 | |
| 28 | $C_{14}H_{11}N_3O_2$ | 235.2 | 66.40<br>66.45 | 4.38<br>4.4 | 16.59<br>16.6 | |
| 29 | $C_{17}H_{17}N_3O_2$ | 295.3 | 69.14<br>69.0 | 5.80<br>5.8 | 14.23<br>14.0 | |
| 30 | $C_{19}H_{21}N_3O_2$ | 323.4 | 70.57<br>70.4 | 6.55<br>6.55 | 12.99<br>12.8 | |
| 31 | $C_{18}H_{19}N_3O_2$ | 309.4 | 69.88<br>69.6 | 6.19<br>6.3 | 13.58<br>13.4 | |
| 32 | $C_{18}H_{19}N_3O_2$ | 309.4 | 69.88<br>69.75 | 6.19<br>6.2 | 13.58<br>13.4 | |

Tableau II (suite)

| Ex No. | Formule | Pds mol. | Calculé/Trouvé | | | |
|---|---|---|---|---|---|---|
| | | | C | H | N | X |
| 33 | $C_{20}H_{23}N_3O_2$ | 337.4 | 71.19<br>71.1 | 6.87<br>6.95 | 12.45<br>12.4 | |
| 34 | $C_{21}H_{25}N_3O_2$ | 351.45 | 71.77<br>71.75 | 7.17<br>7.2 | 11.96<br>11.9 | |
| 35 | $C_{19}H_{21}N_3O_2$ | 323.4 | 70.57<br>70.4 | 6.55<br>6.55 | 12.99<br>13.0 | |
| 36 | $C_{20}H_{23}N_3O_2$ | 337.4 | 71.19<br>70.9 | 6.87<br>6.8 | 12.45<br>12.5 | |
| 37 | $C_{20}H_{21}N_3O_2$ | 335.4 | 71.62<br>71.7 | 6.31<br>6.3 | 12.53<br>12.5 | |
| 38 | $C_{16}H_{15}N_3O_2$ | 281.3 | 68.31<br>68.1 | 5.37<br>5.4 | 14.94<br>15.0 | |
| 39 | $C_{19}H_{21}N_3O_2$ | 323.4 | 70.57<br>70.2 | 6.55<br>6.4 | 12.99<br>13.0 | |
| 40 | $C_{17}H_{17}N_3O_2$ | 295.4 | 69.14<br>69.0 | 5.80<br>5.8 | 14.23<br>14.2 | |
| 41 | $C_{18}H_{19}N_3O_2$ | 309.4 | 69.88<br>69.8 | 6.19<br>6.2 | 13.58<br>13.6 | |
| 42 | $C_{15}H_{13}N_3O_2$ | 267.3 | 67.41<br>67.15 | 4.90<br>4.9 | 15.72<br>15.6 | |

Tableau II (suite)

| Ex No. | Formule | Pds mol. | Calculé/Trouvé | | | |
|---|---|---|---|---|---|---|
| | | | C | H | N | X |
| 43 | $C_{18}H_{19}N_3O_2$ | 309.4 | 69.88<br>69.5 | 6.19<br>6.2 | 13.58<br>13.4 | |
| 44 | $C_{19}H_{19}N_3O_2$ | 321.4 | | | | |
| 45 | $C_{20}H_{23}N_3O_2$ | 337.4 | 71.19<br>71.2 | 6.87<br>6.8 | 12.45<br>12.4 | |
| 46 | $C_{21}H_{25}N_3O_2$ | 351.45 | 71.77<br>71.8 | 7.17<br>7.2 | 11.96<br>11.9 | |
| 47 | $C_{20}H_{23}N_3O_2$ | 341.4 | 71.19<br>71.3 | 6.87<br>6.9 | 12.45<br>12.5 | |
| 48 | $C_{19}H_{21}N_3O_2$ | 323.4 | 70.57<br>70.3 | 6.55<br>6.5 | 12.99<br>12.9 | |

**Exemple 49**

6-éthyl 5-méthyl 7-(méthylthio)imidaso/1,2-a/pyrimidin-2-yl)phénylméthanone.

On met en suspension 4,5 g de 5-éthyl 4-méthyl (6-méthylthio) 2-pyrimidinamine dans 28 $cm^3$ d'éther éthylique, puis ajoute 5,5 g de 3-bromo 1-phénylpropane-1,2-dione.

Après deux jours sous agitation à température ambiante, on filtre le sel de pyrimidiniminium formé, le lave à l'éther et le chauffe pendant une heure dans de l'éthanol au reflux. On évapore la solution sous pression réduite et reprend les résidus par un mélange de chloroforme et de solution aqueuse de carbonate de potassium.

On lave la phase organique, la sèche et l'évapore à sec sous pression réduite, on triture le résidu dans l'éthanol, essore les cristaux, les recristallise dans le méthanol et obtient 4,1 g de produit attendu.

**Exemples 50 à 53**

En utilisant une méthode analouge à celle de l'exemple 49 mais en utilisant au départ les composés correspondants de formule II dans laquelle $R_1$, $R_2$, $R_3$ et X ont les significations indiquées dans le tableau III ci-après, on a préparé les composés des exemples 50 à 53(voir tableau III ci-après).

Les analyses spectrométriques, les rendements et le points de fusion de ces composés sont aussi fournis dans le tableau III. Les résultats de la microanalyse sont donnés dans le tableau IV.

**Exemple 50**

5-méthyl 7-(méthylthio) 6-n-propylimidaso/1,2-a/pyrimidin-2-yl)phénylméthanone.

**Exemple 51**

6-allyl 5-méthyl 7-(méthylthio)imidaso/1,2-a/pyrimidin-2-yl)phénylméthanone.

**Exemple 52**

5,6-diéthyl 7-(méthylthio)imidaso/1,2-a/pyrimidin-2-yl)phénylméthanone.

**Exemple 53**

5-éthyl 7-(méthylthio) 6-n-propylimidaso/1,2-a/pyrimidin-2-yl)phénylméthanone.

TABLEAU III

| Ex. No. | R | $R_1$ | $R_2$ | $R_3$-X | Rdt % | IRcm$^{-1}$ (KBr) | F °C |
|---|---|---|---|---|---|---|---|
| 49 | Ph | $CH_3$ | $C_2H_5$ | $CH_3$-S | 53 | 3120,1640 | 182-4 |
| 50 | Ph | $CH_3$ | $C_3H_7$ | $CH_3$-S | 33 | 3120,1640 | 146-7 |
| 51 | Ph | $CH_3$ | $-CH_2-CH=CH_2$ | $CH_3$-S | 45 | 3120,1635 | 167-9 |
| 52 | Ph | $C_2H_5$ | $C_2H_5$ | $CH_3$-S | 22 | 3110,1640 | 142-4 |
| 53 | Ph | $C_2H_5$ | $C_3H_7$ | $CH_3$-S | 24 | 3130,1645 | 156-8 |

TABLEAU IV

| Ex. No. | Formule | Pds mol. | Calculé/Trouvé | | | |
|---|---|---|---|---|---|---|
| | | | C | H | N | S |
| 49 | $C_{17}H_{17}N_3OS$ | 311.4 | 65.57<br>65.5 | 5.50<br>5.6 | 13.49<br>13.45 | 10.30<br>10.2 |
| 50 | $C_{18}H_{19}N_3OS$ | 325.4 | 66.44<br>65.9 | 5.89<br>5.8 | 12.91<br>12.7 | 9.84<br>9.7 |
| 51 | $C_{18}H_{17}N_3OS$ | 323.4 | 66.85<br>66.9 | 5.30<br>5.4 | 12.99<br>12.95 | 9.91<br>10.0 |
| 52 | $C_{18}H_{19}N_3OS$ | 325.4 | 66.44<br>66.1 | 5.89<br>5.9 | 12.91<br>12.7 | 9.84<br>9.7 |
| 53 | $C_{19}H_{21}N_3OS$ | 339.5 | 67.23<br>66.9 | 6.24<br>6.1 | 12.38<br>12.2 | 9.44<br>9.3 |

**Exemple 54**

6-éthyl 7-méthoxy 5-méthylimidaso/1,2-a/pyrimidin-2-yl) (4-méthoxyphényl)méthanone.

a) <u>6-éthyl 7-méthoxy 5-méthylimidaso/1,2-a/pyrimidin-2-carbo-xylate d'éthyle</u>.

On agite à température ambiante pendant 24 heures une solution de 50,1 g de 5-éthyl 4-méthoxy 6-méthyl 2-pyrimidinamine et 70,2 g de bromopyruvate d'éthyle dans 300 cm$^3$ d'éther éthylique. On filtre le précipité formé, le lave à l'éther et le sèche. On le met en suspension dans 250 cm$^3$ d'éthanol puis porte au reflux pendant deux heures.

On refroidit, évapore à sec et ajoute au résidu un mélange de chloroforme et d'une solution aqueuse à 5% de bicarbonate de sodium.

On décante, réextrait la phase aqueuse au chloroforme, réunit les phases organiques, les sèche et évapore le solvant.

On obtient le produit attendu sous forme de cristaux blancs que l'on purifie par chromatographie sur silice en éluant au mélange chloroforme-acétate d'éthyle (7/3).

On obtient 31,4 g de produit que l'on triture dans un mélange acétate d'éthyle-éther.

On filtre, sèche et obtient le produit attendu. F=151-152°C

b) <u>6-éthyl 7-méthoxy 5-méthylimidaso/1,2-a/pyrimidin-2-yl) méthanol</u>

On ajoute en une heure par petites fractions, 11,55 g de borohydrure de lithium, sous agitation, à une solution de 19,7 g de produit obtenu ci-dessus dans 300 cm$^3$ de tétrahydrofuranne anhydre.

On agite à température ambiante pendant 18 heures, détruit l'excès de borohydrure par addition de 300 cm$^3$ d'une solution saturée de chlorure de sodium, agite à nouveau le mélange pendant trente minutes puis décante. On extrait la phase aqueuse au chloroforme, réunit les phases organiques, les sèche et évapore le solvant. On obtient 15,4 g de produit attendu. F=186-188°C.

c) <u>6-éthyl 7-méthoxy 5-méthylimidaso/1,2-a/pyrimidin-2-carboxaldéhyde</u>.

On ajoute 40 g de dioxyde de manganèse activé à une solution de 15,25 g de produit obtenu ci-dessus dans 200 cm$^3$ de chloroforme.

On porte au reflux pendant 18 heures, ajoute 20 g de dioxyde de manganèse activé puis poursuit le reflux pendant encore 4 heures. On filtre le mélange refroidi, évapore le filtrat et obtient 10,9 g de produit attendu.

F = 185-186° C. Analyse: pour $C_{11}$ $H_{13}$ $N_3$ $O_2$ Calculé: C % 60,26 H % 5,98 N % 19,17 Trouvé: 60,00 6,0 19,05.

d) 6-éthyl 7-méthoxy 5-méthylimidaso/1,2-a/pyrimidin-2-yl) 4-méthoxyphényl)méthanol.

On prépare une solution de bromure de 4-méthoxy phényl magnésium en traitant 0,54 g de tournure de magnésium avec 4,20 g de para-bromoanisole dans 25 cm³ de tétrahydrofuranne anhydre. On ajoute cette solution goutte à goutte à une solution sous agitation de 1,64 g du produit obtenu au stade c) dans 10 cm³ de tétrahydrofuranne anhydre. On agite à température ambiante pendant une heure, verse le mélange sur une solution aqueuse saturée de chlorure d'ammonium, décante, extrait la phase aqueuse au chloroforme, réunit les phases organiques, les sèche et les évapore à sec.

On chromatografie le résidu sur silice en éluant au mélange chloroforme-acétate d'thyle (7/3) et obtient 2,3 g de produit attendu sous forme de cristaux blancs. F = 117-119° C.

e) 6-éthyl 7-méthoxy 5-méthylimidaso/1,2-a/pyrimidin-2-yl) (4-méthoxyphényl)méthanone.

On ajoute 10g de dioxyde de manganèse activé à une solution 2,2 g de produit obtenu au stade d) dans 100 cm³ de chloroforme, puis agite à température ambiante pendant 18 heures. On filtre le mélange, évapore le filtrat, purifie le résidu par chromatografie sur silice en éluant au mélange chloroforme-méthanol (98/2) et obtient 1,3 g de produit attendu. F = 196-197° C.

**Exemples 55 à 76**

En utilisant une méthode analouge à celle décrite à à l'exemple 54, mais en utilisant au départ les composés correspondants de formule II dans laquelle $R_1$, $R_2$, $R_3$ et X ont les significations indiquées dans le tableau V ci-après, on a préparé les composés des exemples 55 à 76.

Les analyses spectrométriques, rendements, points de fusion et microanalyse des composés sont également fournis dans le tableau V.

**Exemple 54**

(6-éthyl 7-méthoxy 5-méthylimidaso/1,2-a/pyrimidin-2-yl) (4-méthoxyphényl)méthanone.

**Exemple 55**

(6-éthyl 7-méthoxy 5-méthylimidaso/1,2-a/pyrimidin-2-yl) (4-chlorophényl)méthanone.

**Exemple 56**

(6-éthyl 7-méthoxy 5-méthylimidaso/1,2-a/pyrimidin-2-yl) (4-méthylphényl)méthanone.

**Exemple 57**

(6-éthyl 7-méthoxy 5-méthylimidaso/1,2-a/pyrimidin-2-yl) (2-chlorophényl)méthanone.

**Exemple 58**

(6-éthyl 7-méthoxy 5-méthylimidaso/1,2-a/pyrimidin-2-yl) (3-méthoxyphényl)méthanone.

**Exemple 59**

(6-éthyl 7-méthoxy 5-méthylimidaso/1,2-a/pyrimidin-2-yl) (4-méthylthiophényl)méthanone.

**Exemple 60**

6-éthyl 7-méthoxy 5-méthylimidaso/1,2-a/pyrimidin-2-yl (2-thiényl)méthanone.

**Exemple 61**

(6-éthyl 7-méthoxy 5-méthylimidaso/1,2-a/pyrimidin-2-yl) (2-pyridinyl)méthanone.

**Exemple 62**

(6-éthyl 7-méthoxy 5-méthylimidaso/1,2-a/pyrimidin-2-yl) (4-fluorophényl)méthanone.

**Exemple 63**

(6-éthyl 7-méthoxy 5-méthylimidaso/1,2-a/pyrimidin-2-yl) /(3-trifluorométhyl)phényl/méthanone.

**Exemple 64**

1-(6-éthyl 7-méthoxy 5-méthylimidaso/1,2-a/pyrimidin-2-yl) éthanone.

**Exemple 65**

1-(6-éthyl 7-méthoxy 5-méthylimidazo/1,2-a/pyrimidin-2-yl) propanone.

**Exemple 66:**

1-(6-éthyl 7-méthoxy 5-méthylimidazo/1,2-a/pyrimidin-2-yl)butanone.

**Exemple 67**

(6 ethyl 7-methoxy 5-methylimidazo/1,2-a/pyrimidin-2-yl)pentanone.

**Exemple 68**

(6-éthyl 7-méthoxy 5-méthylimidazoo/1,2-a/pyrimidin-2-yl)cyclopropyl méthanone.

**Exemple 69**

(6-éthyl 7-méthoxy 5-méthylimidazo/1,2-a/pyrimidin-2-yl) cyclopentyl méthanone.

**Exemple 70**

(6-éthyl 7-méthoxy 5-méthylimidazo/1,2-a/pyrimidin-2-yl) cyclohexyl méthanone.

**Exemple 71**

1-(6-éthyl 7-méthoxy 5-méthylimidazo/1,2-a/pyrimidin-2-yl) 4-penten-1-one.

**Exemple 72**

1-(6-éthyl 7-méthoxy 5-méthylimidazo/1,2-a/pyrimidin-2-yl) 2-cyclohexyl éthanone.

**Exemple 73**

(6-éthyl 7-méthoxy 5-méthylimidazo/1,2-a/pyrimidin-2-yl) 1-naphthyl méthanone.

**Exemple 74**

(6-éthyl 7-méthoxy 5-méthylimidazo/1,2-a/pyrimidin-2-yl) (4-diméthylamino phényl)méthanone.

**Exemple 75**

1-(6-éthyl 7-méthoxy 5-méthylimidazo/1,2-a/pyrimidin-2-yl) 2-méthyl 2-propen-1-one.

**Exemple 76**

1-(6-éthyl 7-méthoxy 5-méthylimidazo/1,2-a/pyrimidin-2-yl) 3-méthyl butanone.

TABLEAU V

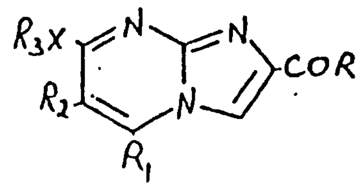

| Ex | R | $R_1$ | $R_2$ | $R_3X$ | Rdt | IR $cm^{-1}$ (KBr) | F | Formule | Pds mol. | Calculé/Trouvé C | H | N | X |
|---|---|---|---|---|---|---|---|---|---|---|---|---|---|
| 54 | 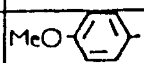 | Me | Et | MeO | 37 | 3150, 1650 | 196-7 | $C_{18}H_{19}N_3O_3$ | 325.4 | 66.45<br>66.4 | 5.89<br>6.0 | 12.91<br>12.8 | |
| 55 | 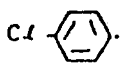 | Me | Et | MeO | 48 | 3120, 1645 | 200-2 | $C_{17}H_{16}ClN_3O_2$ | 329.8 | 61.92<br>62.0 | 4.89<br>4.9 | 12.74<br>12.7 | Cl 10.74<br>10.8 |
| 56 | 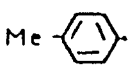 | Me | Et | MeO | 49 | 3090, 1645 | 169-70 | $C_{18}H_{19}N_3O_2$ | 309.4 | 69.88<br>69.9 | 6.19<br>6.25 | 13.58<br>13.55 | |
| 57 | 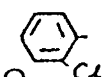 | Me | Et | MeO | 25 | 3140, 1650 | 218-20 | $C_{17}H_{16}ClN_3O_2$ | 329.8 | | | | |
| 58 | 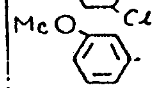 | Me | Et | MeO | 47 | 3170, 1635 | 149-50 | $C_{18}H_{19}N_3O_3$ | 325.4 | 66.45<br>66.5 | 5.89<br>5.95 | 12.91<br>12.8 | |
| 59 | 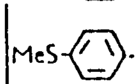 | Me | Et | MeO | 46 | 3130, 1645 | 201-2 | $C_{18}H_{19}N_3O_2S$ | 341.4 | 63.32<br>63.4 | 5.61<br>5.6 | 12.31<br>12.3 | S 9.39<br>9.4 |
| 60 | 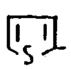 | Me | Et | MeO | 45 | 3130, 1640 | 206-8 | $C_{15}H_{15}N_3O_2S$ | 301.4 | 59.78<br>59.7 | 5.02<br>5.1 | 13.94<br>13.8 | S 10.64<br>S 10.7 |
| 61 | 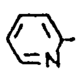 | Me | Et | MeO | 44 | 3180, 1645 | 211-3 | $C_{16}H_{16}N_4O_2$ | 296.3 | 64.85<br>64.7 | 5.44<br>5.5 | 18.91<br>18.85 | |

| Ex | R | $R_1$ | $R_2$ | $R_3X$ | Rdt | IR cm-1 (KBr) | F | Formule | Pds mol. | Calculé/Trouvé | | | |
|---|---|---|---|---|---|---|---|---|---|---|---|---|---|
| | | | | | | | | | | C | H | N | X |
| 62 | 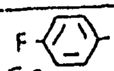 | Me | Et | MeO | 51 | 3120, 1645 | 182-4 | $C_{17}H_{16}FN_3O_2$ | 313.3 | 65.17<br>65.1 | 5.15<br>5.2 | 13.41<br>13.45 | F 6.06<br>6.0 |
| 63 | 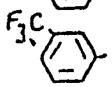 | Me | Et | MeO | 45 | 3130, 1645 | 170-2 | $C_{18}H_{16}F_3N_3O_2$ | 363.4 | 59.50<br>59.35 | 4.44<br>4.55 | 11.56<br>11.5 | F15.69<br>F15.7 |
| 64 | Me | Me | Et | MeO | 45 | 3140, 1685 | 136-7 | $C_{12}H_{15}N_3O_2$ | 233.3 | 61.79<br>61.8 | 6.48<br>6.5 | 18.01<br>18.0 | |
| 65 | Et | Me | Et | MeO | 42 | 3130, 1680 | 184-5 | $C_{13}H_{17}N_3O_2$ | 247.3 | 63.14<br>63.1 | 6.93<br>7.0 | 16.99<br>17.0 | |
| 66 | Pr | Me | Et | MeO | 39 | 3140, 1680 | 150-1 | $C_{14}H_{19}N_3O_2$ | 261.3 | 64.35<br>64.15 | 7.33<br>7.3 | 16.08<br>16.0 | |
| 67 | Bu | Me | Et | MeO | 34 | 3130, 1680 | 157-8 | $C_{15}H_{21}N_3O_2$ | 275.4 | 65.43<br>65.5 | 7.69<br>7.7 | 15.26<br>15.4 | |
| 68 | 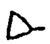 | Me | Et | MeO | 36 | 3130, 1650 | 192-3 | $C_{14}H_{17}N_3O_2$ | 259.3 | 64.85<br>64.8 | 6.61<br>6.6 | 16.20<br>16.2 | |
| 69 | 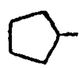 | Me | Et | MeO | 20 | 3130, 1670 | 176-8 | $C_{16}H_{21}N_3O_2$ | 287.4 | 66.88<br>66.8 | 7.37<br>7.3 | 14.62<br>14.5 | |
| 70 | 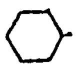 | Me | Et | MeO | 34 | 3130, 1670 | 198-200 | $C_{17}H_{23}N_3O_2$ | 301.4 | 67.75<br>67.8 | 7.69<br>7.7 | 13.94<br>13.9 | |
| 71 | $CH_2=CH(CH_2)_2$ | Me | Et | MeO | 31 | 3130, 1675 | 163-5 | $C_{15}H_{19}N_3O_2$ | 273.3 | 65.91<br>65.6 | 7.01<br>6.9 | 15.37<br>15.3 | |
| 72 | 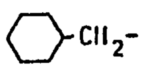 | Me | Et | MeO | 39 | 3140, 1670 | 177-9 | $C_{18}H_{25}N_3O_2$ | 315.4 | | | | |
| 73 | 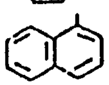 | Me | Et | MeO | 33 | 3140, 1640 | 240-2 | $C_{21}H_{19}N_3O_2$ | 345.4 | | | | |
| 74 | 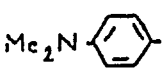 | Me | Et | MeO | 19 | 3130, 1640 | 221-3 | $C_{19}H_{22}N_4O_2$ | 338.4 | 67.44<br>67.5 | 6.55<br>6.6 | 16.56<br>16.55 | |
| 75 | $CH_2=CMe$ - | Me | Et | MeO | 35 | 3130, 1640 | 148-50 | $C_{14}H_{17}N_3O_2$ | 259.3 | 64.85<br>64.7 | 6.61<br>6.6 | 16.20<br>16.1 | |
| 76 | $Me_2CHCH_2$- | Me | Et | MeO | 35 | 3130, 1675 | 139-40 | $C_{15}H_{21}N_3O_2$ | 275-4 | 65.43<br>65.65 | 7.69<br>7.6 | 15.26<br>15.25 | |

**Exemple 77:**

On a préparé des comprimés correspondants à la formulation suivante:
- composés de l'exemple I............................ 20 mg
- Excipient q.s. pour un comprimé terminé à...........150 mg.
(Détail de l'excipient: lactose, amidon, talc, stéarate de magnésium).

**Exemple 78**

On a préparé des comprimés correspondants à la formulation suivante:
- composés de l'exemple 49........................... 20 mg
- Excipient q.s.pour un comprimé terminé à........... 150 mg
(Détail de l'excipient: lactose, amidon, talc, stéarate de magnésium).

**Activités biochimigue et pharmacologique**

**1) Activité biochimique**

L'affinité des composés pour les récepteurs desbenzodiazépines a été évaluée en utilisant un radioligand /$^3$H/ flunitrazépam et la méthode de Squires et Braestrup (Nature, 1977, 266,732) modifiée.
Les valeurs indiquées dans 1e tableau VI ci-après sont les concentrations nanomolaires du produit testé qui inhibent dans une proportion de 50 % la liaison spécifique de 0,6 nanomoles de /$^3$H/ flunitrazépam dans despréparations de membranes de cerveaux antérieurs de rats ($CI_{50}$) en nanomoles.

**2) Activité pharmacologique**

L'étude de 1'activité anxiolytique des composés a été effectuée par une méthode selon Geller et Seifter (Psychopharmacologia, 1960, I, 482) modifiée.
Les valeurs données dans le tableau VI sont les doses efficaces minimum auxquelles il est observé un accroissement des chocs par rapport aux témoins (DEM mg/kg p.o).

TABLEAU VI

| Exemple | Liaison aux récepteurs ($IC_{50}$nM) | Méthode de Geller (DEM mg/kg po) |
|---|---|---|
| 1 | 61 | 5 |
| 3 | 800 | 5 |
| 4 | 200 | 20 |
| 6 | 35 | 2 |
| 7 | 235 | 50 |
| 8 | 125 | 10 |
| 9 | 45 | >50 |
| 10 | 4000 | 5 |
| 11 | 37 | 2 |
| 12 | 8.5 | 10 |
| 13 | 1850 | 50 |
| 14 | 535 | 1 |
| 15 | 295 | 2 |
| 16 | 56 | 2 |
| 17 | 135 | 10 |
| 18 | 76 | 50 |
| 19 | 180 | 20 |
| 20 | 45 | 4 |
| 21 | 190 | 50 |
| 22 | 89 | 50 |
| 23 | 30 | 10 |
| 24 | 250 | 50 |
| 25 | 1900 | 50 |
| 26 | 740 | 20 |
| 27 | 470 | 50 |
| 28 | 8500 | >50 |
| 29 | 1000 | 20 |
| 30 | 42 | 2 |
| 31 | 107 | 2 |
| 32 | 85 | 5 |
| 33 | 1250 | >50 |

TABLEAU VI (Suite)

| Exemple | Liaison aux récepteurs ($IC_{50}$nM) | Méthode de Geller (DEM mg/kg po) |
|---|---|---|
| 34 | 710 | 50 |
| 35 | 34 | 10 |
| 36 | 34 | 10 |
| 37 | 135 | 50 |
| 38 | 1500 | 5 |
| 39 | 400 | $>$ 50 |
| 40 | 1900 | 50 |
| 41 | 300 | 50 |
| 42 | 5900 | 50 |
| 43 | 340 | 10 |
| 44 | 42 | 10 |
| 45 | 500 | $>$ 50 |
| 46 | 1200 | $>$ 20 |
| 47 | 107 | 20 |
| 48 | 400 | 20 |
| 49 | 12 | 2 |
| 50 | 19 | 50 |
| 51 | 8 | 5 |
| 52 | 12 | 50 |
| 53 | 47 | 10 |
| 54 | 59 | 10 |
| 55 | 85 | 50 |
| 56 | 68 | 50 |
| 57 | 100 | 50 |
| 58 | 500 | 50 |
| 59 | 300 | 50 |
| 60 | - | 50 |
| 61 | - | 50 |
| 62 | - | 10 |
| 63 | - | 50 |
| 64 | - | 50 |

**Revendications**

pour les Etats contractants: BE, CH, DE, FR, IT, LI, LU, NL, SE

1) Nouvelles imidazo/1,2-a/ pyrimidines répondant à la formule (I):

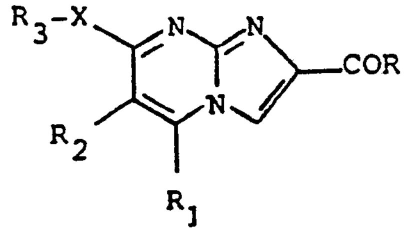

(I)

dans laquelle R représente un radical alcoyle linéaire renfermant de 1 à 8 atomes de carbone, un radical alcényle linéaire renfermant de 2 à 8 atomes de carbone, un radical cycloalcoyle renfermant de 3 à 7 atomes de carbon; un radical (cycloalcoyl)alcoyle renfermant de 4 à 12 atomes de carbone, un radical aryle renfermant de 6 à 12 atomes de carbone ou un radical thiényle ou pyridyle, chacun des radicaux ci-dessus pouvant être substitué par un ou plusieurs atomes d'halogènes, un ou plusieurs radicaux alcoyles alcoylthio ou alcoxy renfermant de 1 à 5 atomes de carbone, un ou plusieurs radicaux amino, alcoylamino ou dialcoylamino, dans lesquels les radicaux alcoyles renfermant de 1 à 5 atomes de carbone, ou par un ou plusieurs radicaux trifluorométhyles;

$R_1$ et $R_2$, identiques ou différents, représentent un atome d'hydrogène, un radical alcoyle linéaire renfermant de 1 à 8 atomes de carbone, un radical alcényle linéaire renfermant de 2 à 5 atomes de carbone, un radical cycloalcoyle renfermant de 3 à 7 atomes de carbone, un radical (cycloalcoyl)alcoyle renfermant de 4 à 12 atomes de carbone, un radical benzyle ou un radical phénéthyle, ou $R_1$ et $R_2$ forment ensemble un radical alcoylène linéaire renfermant de 3 à 5 atomes de carbone, chacun des radicaux ci-dessus pouvant être substitué par un ou plusieurs atomes d'halogènes, un ou plusieurs radicaux alcoyles, alcoxy ou alcoylthio renfermant de 1 à 5 atomes de carbone, par un ou plusieurs radicaux amino, alcoylamino ou dialcoylamino dans lesquels les radicaux alcoyles renfermant de 1 à 5 atomes de carbone, ou par un ou plusieurs radicaux trifluorométhyles, X représente un atome d'oxygène ou de soufre et $R_3$ représente un radical alcoyle renfermant de 1 à 5 atomes de carbone, ainsi que leurs sels d'addition avec les acides minéraux ou organiques.

2) Nouvelles imidazo/1,2-a/pyrimidines telles que définies à la revendication 1, et leurs sels, caractérisées en ce que dans ladite formule (I), R représente un radical phényle, $R_1$,, $R_2$, $R_3$ et X ont la signification déjà indiquée.

3) Nouvelles imidazo /1,2-a /pyrimidines, telles que définies à la revendication 1 ou 2, et leurs sels, caractérisées en ce que dans ladite formule (I), $R_1$ et $R_2$, identiques ou différents, représentent un atome d'hydrogène, un radical méthyle, éthyle, propyle, isopropyle, hexyle, allyle, benzyle ou $R_1$ et $R_2$ forment ensemble un radical 1,3-propylène, 1,4-butylène, 1,5-pentylène ou un radical $-CH_2-$

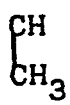

$-CH_2-CH_2-$, $R_3$ représente un radical méthyle ou éthyle, R et X ayant la signification déjà indiquée.

4) La /6-éthyl-5-méthyl-7-(méthylthio)imidazo/1,2-a/pyrimidin-2-yl/phénylméthanone et ses sels.

5) L'un quelconque des produits de formule I dont les noms suivent:

- la (5-méthoxy 6,7,8,9-tétrahydroimidazo/1,2-a/quinazolin-2-yl)phénylméthanone,
- la /5-(méthylthio)cyclopenta /e/imidazo/1,2-a/pyrimidin-2-yl/ phénylméthanone,
- la /7-méthoxy 5-méthyl 6-(1-méthyl éthyl)imidazo/1,2-a/pyri-midin-2-yl/phénylméthanone et
- la (6-éthyl-7-méthoxy 5-méthyl imidazo/1,2-a/pyrimidin-2-yl) phényl méthanone ainsi que leurs sels.

6) Procédé de préparation des nouvelles imidazo/1,2-a/pyrimidines telles que définies par la formule (I) de la revendication ainsi que de leurs sels, caractérisé en ce que l'on fait réagir un produit de formule (II):

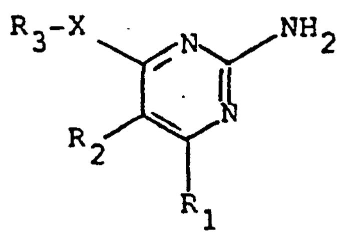

(II)

dans laquelle $R_1$, $R_2$, $R_3$ et X ont la signification déjà indiquée, avec un produit de formule (III):

$Y-CH_2-CO-CO-R$ (III)

dans laquelle R a la signification déjà indiquée et Y est un groupement pouvant former par élimination un anion $Y^{\ominus}$ pour obtenir un produit de formule (IV):

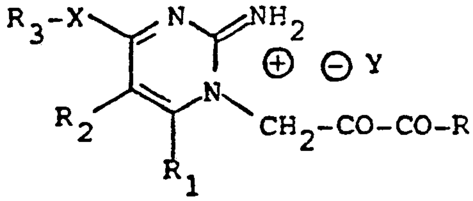

(IV)

dans laquelle R, $R_1$, $R_2$, $R_3$, X et Y ont la signification déjà indiquée puis cyclise ledit produit de formule (IV) obtenu pour obtenir un produit de formule (I) que l'on peut salifier le cas échéant.

7) Procédé de préparation selon la revendication 6, caractérisé en ce que:

a) la réaction du produit de formule (II) avec le produit de formule (III) est effectuée au sein d'un solvant organique tel que l'éther éthylique, le tétrahydrofuranne ou le diméthoxyéthane;

b) la cyclisation du produit de formule (IV) avec ou sans isolement intermédiaire est effectuée au reflux du mélange réactionnel au sein d'un solvant tel que le méthanol ou par agitation à température ambiante, au sein d'un solvant tel que le méthanol, en présence d'une base telle que le carbonate de potassium.

8) Variante du procédé de préparation des produits de formule I, ainsi que de leurs sels, caractérisée en ce que l'on fait réagir un produit de formule (II):

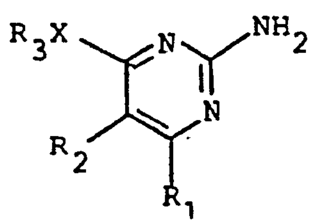

(II)

dans laquelle $R_1$, $R_2$, $R_3$ et X ont la signification déjà indiquée, avec un produit de formule:

$A\text{-}CH_2\text{-}CO\text{-}CO_2Q$

dans laquelle A représente un groupement nucléophile et Q represente un groupement estérifiant, cyclise le produit obtenu de formule:

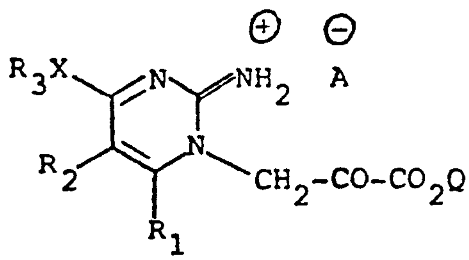

dans laquelle $R_1$, $R_2$, $R_3$, Q et X ont la signification déjà indiquée et $A^{\ominus}$ représente un anion dérivé de A, pour obtenir le produit de formule IX:

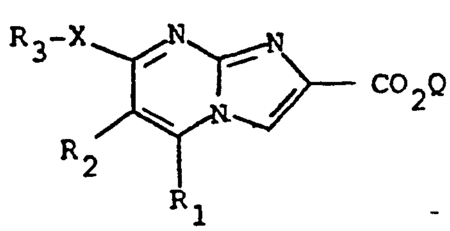

(IX)

dans laquelle $R_1$, $R_2$, $R_3$, X et Q ont la signification déjà indiquée, réduit le produit de formule IX pour obtenir le produit de formule VIII:

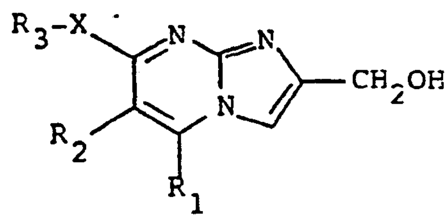

(VIII)

dans laquelle $R_1$, $R_2$, $R_3$ et X ont la signification déjà indiguée oxyde le produit de formule VIII pour obtenir le produit de formule VI:

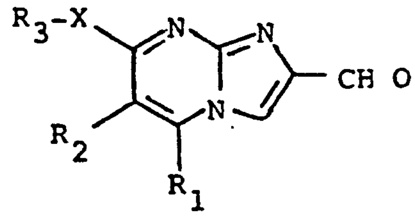

(VI)

dans laquelle $R_1$, $R_2$, $R_3$ et X ont la signification dejà indiquee, fait réagir le produit de formule VI avec un dérivé magnésien d'un produit de formule VII:

R-K (VII)

dans laquelle R a la signification déjà indiquée et K représente un atome de lithium ou un reste -MgHal, dans lequel Hal represente un atome de chlore, de brome ou d'iode, pour obtenir un produit de formule V:

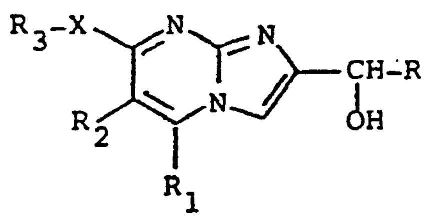

(V)

dans laquelle R, $R_1$, $R_2$, $R_3$ et X ont la signification déja indiquée, puis oxyde le produit de formule V, pour obtenir le produit de formule I attendu que l'on peut salifier, le cas échéant.

9) Procédé de préparation selon la revendication 8, caractérisé en ce que:

a) la cyclisation menant au produit de formule IX est effectuée dans les mêmes conditions que celles du produit de formule IV indiquée à la revendication 7;

b) Q est un radical alcoyle ou aralcoyle et plus particulièrement un radical alcoyle renfermant de 1 à 3 atomes de carbone tel qu'un radical éthyle;

c) la réduction du produit de formule IX est effectuée par l'hydrure de lithium-aluminium ou encore par le borohydrure de sodium en présence de chlorure d'aluminium ou le borohydrure de lithium;

d) l'oxydation du produit de formule VIII est effectuée à l'aide de dioxyde de manganèse ou dans les conditions indiquées ciaprès pour l'oxydation du produit de formule V;

e) la réaction du produit de formule VI avec le produit de formule VII est effectuée dans des conditions anhydres dans un solvant organique tel que le tétrahydrofuranne;

f) l'oxydation du produit de formule V est effectuée avec le dioxyde de manganèse, l'acide nitrique, le chlorure ferrique ou l'oxyde de chrome en présence de pyridine, ou encore par la méthode d'Oppenauer ou enfin par déshydrogénation en présence d'un catalyseur à base de cuivre.

10) Médicaments, caractérisés en ce qu'ils sont constitués par les nouvelles imidazo/l,2-a/pyrimidines telles que définies par la formule (I) de la revendication 1, ainsi que par leurs sels pharmaceutiquement acceptables.

11) Médicaments caractérisés en ce qu'ils sont constitués par les nouvelles imidazo/1,2-a/pyrimidines telles que définies à l'une quelconque des revendications 2, 3, 4 ou 5, ainsi que par leurs sels pharmaceutiquement acceptables.

12) Compositions pharmaceutiques, caractérisées en ce qu'elles renferment à titre de principe actif, l'un au moins des médicaments tels que définis à l'une quelconque des revendications 10 ou 11.

13) A titre d'intermédiaires nécessaires à la préparation des produits de formule I, telle que définie à la

revendication 1, les produits de formule IV, telle que définie à la revendication 6.

14) A titre d'intermédiaires nécessaires à la préparation des produits de formule (I), telle que définie à la revendication 1, les produits de formule:

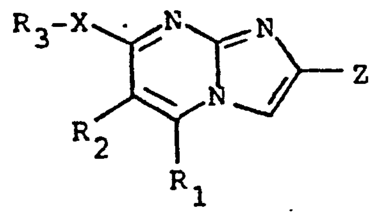

dans laquelle $R_1$, $R_2$, $R_3$ et X sont définis comme précédemment et Z représente un radical -CHO ou

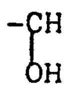

-R¹ dans lequel R' représente un atome d'hydrogène ou un radical R tel que défini précédemment.

**Revendication**

pour l'Etat contractant: AT

1) Procédé de préparation de nouvelles imidazo/1,2-a/pyrimidines répondant à la formule (I):

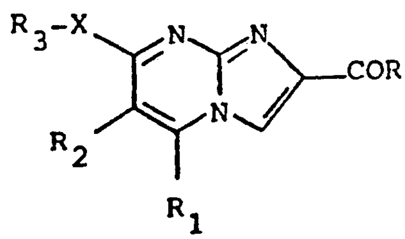

(I)

dans laquelle R représente un radical alcoyle, linéaire renfermant de 1 à 8 atomes de carbone, un radical alcényle, linéaire renfermant de 2 à 8 atomes de carbone, un radical cycloalcoyle renfermant de 3 à 7 atomes de carbone, un radical (cycloalcoyl) alcoyle renfermant de 4 à 12 atomes de carbone, un radical aryle renfermant de 6 à 12 atomes de carbone ou un radical thiényle ou pyridyle, chacun des radicaux ci-dessus pouvant être substitué par un ou plusieurs atomes d'halogènes, un ou plusieurs radicaux alcoyle, alcoylthio ou alcoxy renfermant de 1 à 5 atomes de carbone, un ou plusieurs radicaux amino, alcoylamino ou dialcoylamino, dans lesquels les radicaux alcoyles renfermant de 1 à 5 atomes de carbone, ou par un ou plusieurs radicaux trifluorométhyle;

$R_1$ et $R_2$, identiques ou différents, représentent un atome d'hydrogène, un radical alcoyle, linéaire renfermant de 1 à 8 atomes de carbone, un radical alcényle, linéaire renfermant de 2 à 5 atomes de carbone, un radical cycloalcoyle renfermant de 3 à 7 atomes de carbone, un radical (cycloalcoyl)alcoyle renfermant de 4 à 12 atomes de carbone, un radical benzyle ou un radical phénéthyle ou $R_1$ et $R_2$ forment ensemble un radical alcoylène, linéaire renfermant de 3 à 5 atomes de carbone, chacun des radicaux ci-dessus pouvant être substitué par un ou plusieurs atomes d'halogènes, un ou plusieurs radicaux alcoyles, alcoxy ou alcoylthio renfermant de 1 à 5 atomes de carbone, par un ou plusieurs radicaux amino, alcoylamino ou dialcoylamino dans lesquels les radicaux alcoyles renfermant de 1 à 5 atomes de carbone, ou par un ou plusieurs radicaux trifluorométhyle;

X représente un atome d'oxygène ou de soufre et $R_3$ représente un radical alcoyle renferment de 1 à 5 atomes de carbone, ainsi que de leurs sels d'addition avec les acides minéraux ou organiques caractérisé en ce que:

-<u>soit</u> l'on fait réagir un produit de formule (II):

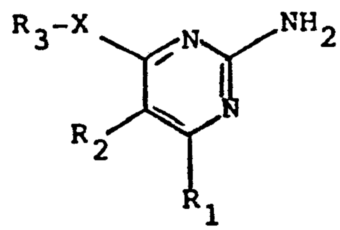

(II)

dans laquelle $R_1$, $R_2$, $R_3$ et X ont la signification déjà indiquée, avec un produit de formule (III):

$Y-CH_2-CO-CO-R$ (III)

dans laquelle R a la signification déjà indiquée et Y est un groupement pouvant former par élimination, un anion $Y^{\ominus}$ pour obtenir un produit de formule (IV):

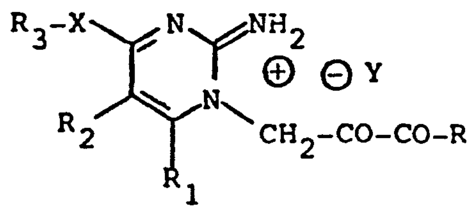

(IV)

dans laquelle R, $R_1$, $R_2$, $R_3$, X et Y ont la signification déjà indiquée, puis cyclise ledit produit de formule (IV) obtenu pour obtenir un produit de formule (I) que l'on peut salifier le cas échéant,

- soit l'on fait réagir un produit de formule (II):

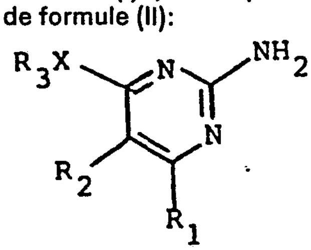

(II)

$NH_2$ dans laquelle $R_1$, $R_2$, $R_3$ et X ont la signification déjà indiquée, avec un produit de formule:

$A-CH_2-CO-CO_2Q$

dans laquelle A représente un groupement nucléophile et Q représente un groupement estérifiant, cyclise le produit obtenu de formule:

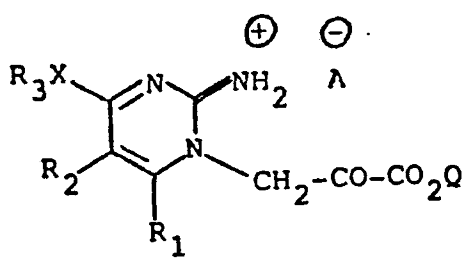

dans laquelle $R_1$, $R_2$, $R_3$, Q et X ont la signification déjà indiquée et $A^{\ominus}$ représente un anion dérivé de A, pour obtenir le produit de formule (IX):

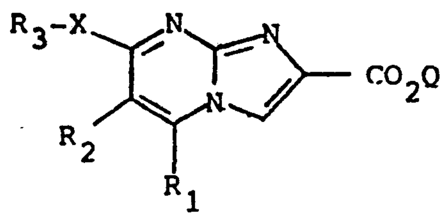

(IX)

dans laquelle $R_1$, $R_2$, $R_3$, X et Q ont la signification déjà indiquée, réduit le produit de formule (IX), pour

obtenir le produit de formule (VIII):

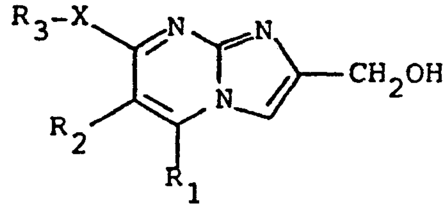

(VIII)

dans laquelle $R_1$, $R_2$, $R_3$ et X ont la signification déjà indiquée, oxyde le produit de formule (VIII), pour obtenir le produit de formule (VI):

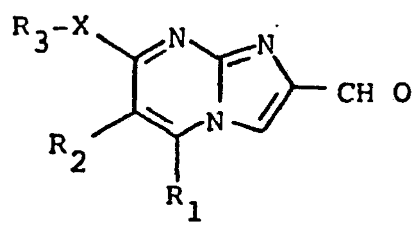

(VI)

dans laquelle $R_1$, $R_2$, $R_3$ et X ont la signification déjà indiquée fait réagir le produit de formule (VI) avec un dérivé magnésien d'un produit de formule (VII):

R-K (VII)

dans laquelle R a la signification déjà indiquée et K represente un atome de lithium ou un reste -MgHal, dans lequel Hal représente un atome de chlore, de brome ou d'iode, pour obtenir un produit de formule (V):

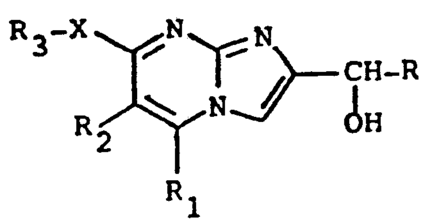

(V)

dans laquelle R, $R_1$, $R_2$, $R_3$ et X ont la signfication déjà indiquée, puis oxyde le produit de formule (V), pour obtenir le produit de formule I attendu que l'on peut salifier, le cas échéant.

2) Procédé selon la revendication 1, pour la préparation des produits de formule I, telle que défânie à la revendication 1 ainsi que de leurs sels, caractérisé en ce que l'on fait réagir un produit de formule (II):

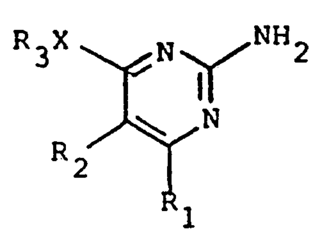

(II)

dans laquelle $R_1$, $R_2$, $R_3$ et X ont la signification déjà indiquée, avec un produit de formule (III):

$Y-CH_2-CO-CO-R$ (III)

dans laquelle R a la signification déjà indiquée et Y est un groupement pouvant former par élimination un anion $Y^{\ominus}$, pour obtenir un produit de formule (IV):

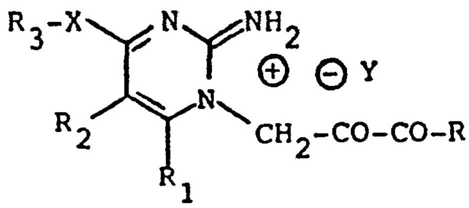

(IV)

dans laquelle R, $R_1$, $R_2$, $R_3$, X et Y ont la signification déjà indiquée, puis cyclise ledit produit de formule (IV) obtenu pour obtenir un produit de formule (I) que l'on peut salifier le cas échéant.

3) Procédé de préparation selon la revendication 1, caractérisé en ce que:

a) la réaction du produit de formule (II) avec le produit de formule (III) est effectuée au sein d'un solvant organique tel que l'éther éthylique, le tétrahydrofuranne ou le diméthoxyéthane;

b) la cyclisation du produit de formule (IV) avec ou sans isolement intermédiaire est effectuée au reflux du mélange réactionnel au sein d'un solvant tel que le méthanol ou par agitation à température ambiante, au sein d'un solvant tel que le méthanol, en présence d'une base telle que le carbonate de potassium.

4) Procédé selon la revendication 1, pour la préparation des produits de formule (I), telle que définie à la revendication I ainsi que de leurs sels, caractérisé en ce que:

l'on fait réagir un produit de formule (II):

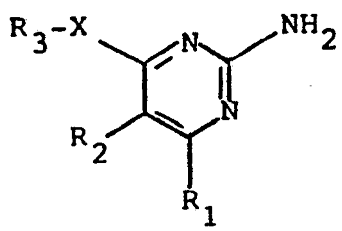

(II)

$NH_2$ dans laquelle $R_1$, $R_2$, $R_3$ et X ont la signification déjà indiquée, avec un produit de formule:

$A\text{-}CH_2\text{-}CO\text{-}CO_2Q$

dans laquelle A représente un groupement nucléophile et Q représente un groupement estérifiant, cyclise le produit obtenu de formule:

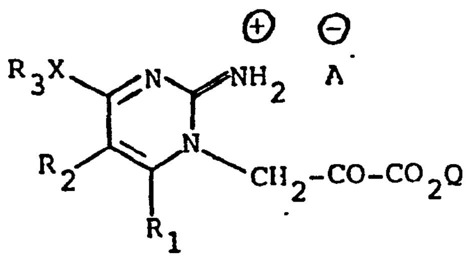

dans laquelle $R_1$, $R_2$, $R_3$, Q et X ont la signification deja indiquée et $A^{\ominus}$, représente un anion dérivé de A, pour obtenir le produit de formule (IX):

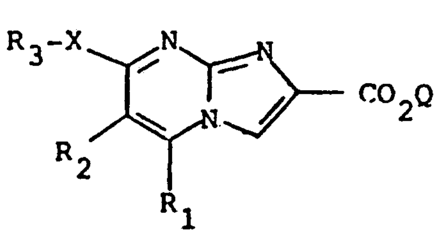

(IX)

dans laquelle $R_1$, $R_2$, $R_3$, X et Q ont la signification déjà indiquée, réduit le produit de formule (IX) pour obtenir le produit de formule (VIII):

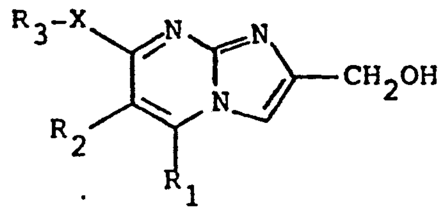

(VIII)

dans laquelle $R_1$, $R_2$, $R_3$ et X ont la signification déjà indiquée, oxyde le produit de formule (VIII) pour obtenir le produit de formule (VI):

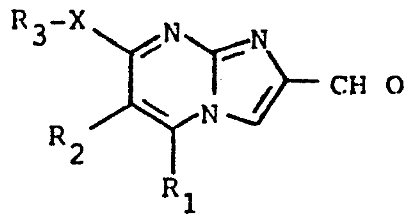

(VI).

dans laquelle $R_1$, $R_2$, $R_3$ et Y ont la signification déjà indiquée, fait réagir le produit de formule (VI) avec un dérivé magnésien d'un produit de formule (VII):

R-K (VII)

dans laquelle R a la signification déjà indiquée et K représente un atome de lithium ou un reste -MgHal dans lequel Hal représent un atome de chlore, de brome ou d'iode, pour obtenir un produit de formule (V):

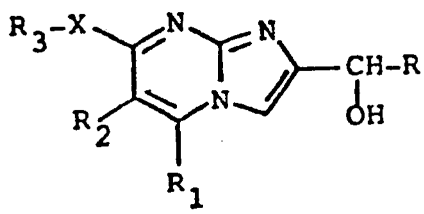

(V)

dans laquelle R, $R_1$, $R_2$, $R_3$ et X ont la signification déjà indiquée, puis oxyde le produit de formule (V) pour obtenir le produit de formule (I) attendu que l'on peut salifier, le cas échéant.

5) Procédé de préparation selon la revendication 4, caractérisé en ce que:

a) la cyclisation menant au produit de formule (IX) est effectuée dans les mêmes conditions que celles du produit de formule (IV) indiquée à la revendication 7,

b) Q est un radical alcoyle ou aralcoyle et plus particulièrement un radical alcoyle renfermant de 1 à 3 atomes de carbone tel qu'un radical éthyle;

c) la réduction du produit de formule (IX) est effectuée par l'hydrure de lithium-aluminium ou encore par le borohydrure de sodium en présence de chlorure d'aluminium ou le borohydrure de lithium;

d) l'oxydation du produit de formule (VIII) est effectuée à l'aide de dioxyde de manganèse ou dans les conditions indiquées ciaprès pour l'oxydation du produit de formule (V);

e) la réaction du produit de formule (VI) avec le produit de formule (VII) est effectuée dans des conditions anhydres dans un solvant organique tel que le tétrahydrofuranne;

f) l'oxydation du produit de formule (V) est effectuée avec le dioxyde de manganèse, l'acide nitrique, le chlorure ferrique ou l'oxyde de chrome en présence de pyridine, ou encore par la méthode d'Oppenauer ou enfin par déshydrogénation en présence d'un catalyseur à base de cuivre,

6) Procédé selon l'une quelconque des revendications 1 à 5, caractérisé en ce que l'on utilise au départ un produit de formule (III) ou (VII) dans laquelle R représente un radical phényle.

7) Procédé selon l'une quelconque des revendications 1 à 6, caractérisé en ce que l'on utilise au départ un produit de formule (II) dans laquelle $R_1$ et $R_2$, identigues ou différents, représentent un atome d'hydrogène, un radical méthyle, éthyle, propyle, isopropyle, hexyle, allyle, benzyle ou $R_1$ et $R_2$ forment ensemble un radical 1,3-propylène, 1,4-butylène, 1,5-pentylène ou un radical $-CH_2-$

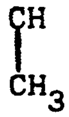

$-CH_2-CH_2-$,

$R_3$ représente un radical méthyle ou éthyle, R et X ayant la signification déjà indiquée.

8) Procédé selon l'une quelconque des revendications 1 à 7, caractérisé en ce que l'on met en oeuvre au départ des produits choisis de manière telle que l'on prépare la /6-éthyl 5-méthyl 7-(méthylthio)imidazo/1,2-a/pyrimidin-2-yl/phényl méthanone et ses sels.

9) Procédé selon l'une quelconque des revendications 1 à 7, caractérisé en ce cue l'on met en oeuvre au départ des produits choisis de manière telle que l'on prépare l'un quelconque des produits de formule I dont les noms suivent:

- la (5-méthoxy 6,7,8,9-tétrahydroimidazo/1,2-a/quinazolin-2-yl)phénylméthanone,
- la /5-(méthylthio)cyclopenta /e/imidazo/1,2-a/pyrimidin-2-yl/phénylméthanone,
- la /7-méthoxy 5-méthyl 6-(1-méthyl éthyl)imidazo/1,2-a/ pyrimidin-2-yl/phénylméthanone et
- la (6-éthyl 7-méthoxy 5-méthylimidazo/1,2-a/pyrimidin-2-yl) phényl méthanone ainsi que leurs sels.

**Patentansprüche**

für die Vertragsstaaten: BE, CH, DE, FR, IT, LI, LU, NL, SE

1. Neue Imidazo[1.2-a]pyrimidine der Formel (I)

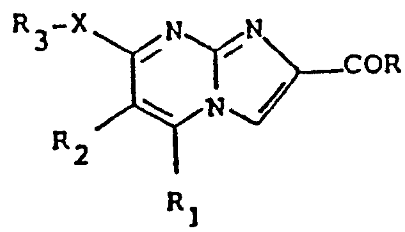

(I)

worin R einen linearen Alkylrest mit 1 bis 8 Kohlenstoffatomen, einen linearen Alkenylrest mit 2 bis 8 Kohlenstoffatomen, einen Cycloalkylrest mit 3 bis 7 Kohlenstoffatomen, einen (Cycloalkyl)-alkylrest mit 4 bis 12 Kohlenstoffatomen, einen Arylrest mit 6 bis 12 Kohlenstoffatomen oder einen Thienyl- oder Pyridylrest bedeutet, wobei ein jeder der vorstehenden Reste durch ein oder mehrere Halogenatome, einen oder mehrere Alkyl-, Alkylthio- oder Alkoxyreste mit 1 bis 5 Kohlenstoffatomen, einen oder mehrere Amino-, Alkylamino- oder Dialkylaminoreste, worin die Alkylreste 1 bis 5 Kohlenstoffatome enthalten, oder durch einen oder mehrere Trifluormethylreste substituiert sein kann,

$R_1$ und $R_2$, die gleich oder verschieden sind, ein Wasserstoffatom, einen linearen Alkylrest mit 1 bis 8 Kohlenstoffatomen, einen linearen Alkenylrest mit 2 bis 5 Kohlenstoffatomen, einen Cycloalkylrest mit 3 bis 7 Kohlenstoffatomen, einen (Cycloalkyl)-alkylrest mit 4 bis 12 Kohlenstoffatomen, einen Benzylrest oder einen Phenethylrest bedeuten oder R und R gemeinsam einen linearen Alkylenrest mit 3 bis 5 Kohlenstoffatomen bilden, wobei ein jeder der vorstehenden Reste durch ein oder mehrere Halogenatome, einen oder mehrere Alkyl-, Alkoxy- oder Alkylthioreste mit 1 bis 5 Kohlenstoffatomen, durch einen oder mehrere Amino-, Alkylamino- oder Dialkylaminoreste, worin die Alkylreste 1 bis 5 Kohlenstoffatome enthalten, oder durch einen oder mehrere Trifluormethylreste substituiert sein kann, X ein Sauerstoff- oder Schwefelatom bedeutet und R einen Alkylrest mit 1 bis 5 Kohlenstoffatomen bedeutet,

sowie deren Additionssalze mit Mineral- oder organischen Säuren.

2. Neue Imidazo[1.2-a]pyrimidine gemäß Anspruch 1 und deren Salze, dadurch gekennzeichnet, daß in der Formel (I) R einen Phenylrest bedeutet, $R_1$, $R_2$ und $R_3$ und X die angegebene Bedeutung besitzen.

3. Neue Imidazo[1.2-a]pyrimidine gemäß Anspruch 1 oder 2 und deren Salze, dadurch gekennzeichnet, daß in der Formel (I) $R_1$ und $R_2$, die gleich oder verschieden sind, ein Wasserstoffatom, einen Methyl-, Ethyl-, Propyl-, Isopropyl-, Hexyl-, Allyl- oder Benzylrest bedeuten oder $R_1$ und $R_2$ gemeinsam einen 1,3-Propylen-, 1,4-Butylen-, 1,5-Pentylenrest oder einen Rest

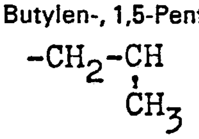

$-CH_2-CH_2-$ bilden, $R_3$ einen Methyl- oder Ethylrest bedeutet, wobei R und X die angegebene Bedeutung besitzen.

4. [6-Ethyl-5-methyl-7-(methylthio)-imidazo[1.2-a]-pyrimidin-2-yl]-phenylmethanon und dessen Salze.

5. Eines der Produkte der Formel I mit den folgenden Bezeichnungen:

(5-Methoxy-6,7,8,9-tetrahydro-imidazo[1.2-a]-chinazolin-2-yl)-phenylmethanon, [5-(Methylthio)-cyclopent-[e]-imidazo[1.2-a] pyrimidin-2-yl]-phenylmethanon,
[7-Methoxy-5-methyl-6-(1-methylethyl)-imidazo-[1.2-a]pyrimidin-2-yl]-phenylmethanon und
(6-Ethyl-7-methoxy-5-methyl-imidazo[(1.2-a]pyrimi-din-2-yl)-phenylmethanon sowie deren Salze.

6. Verfahren zur Herstellung neuer Imidazo[1.2-a]-pyrimidine der Formel (I) gemäß Anspruch 1 sowie von deren Salzen, dadurch gekennzeichnet, daß man ein Produkt der Formel (II)

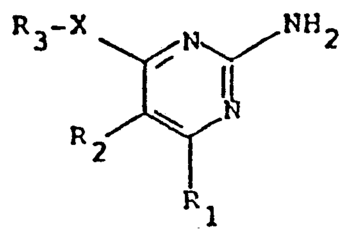

(II)

worin $R_1$, $R_2$, $R_3$ und X die angegebene Bedeutung besitzen, mit einem produkt der Formel (III)
$Y-CH_2-CO-CO-R$ (III)
worin R die angegebene Bedeutung besitzt und Y eine Gruppe darstellt, die durch Eliminierung ein Anion $Y^-$ bilden kann, umsetzt, um ein Produkt der Formel (IV)

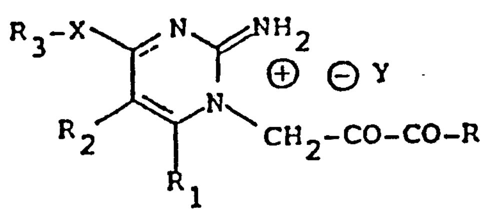

(IV)

zu erhalten, worin R, $R_1$, $R_2$, $R_3$, X und Y die angegebene Bedeutung besitzen, und danach das erhaltene Produkt der Formel (IV) cyclisiert, um ein Produkt der Formel (I) zu erhalten das man gegebenenfalls in ein Salz überführen kann.

7. Verfahren gemäß Anspruch 6, dadurch gekennzeichnet, daß
(a) die Umsetzung des Produkts der Formel (II) mit dem Produkt der Formel (III) in dem Medium eines organischen Lösungsmittels, wie Ethylether, Tetrahydrofuran oder Dimethoxyethan, durchgeführt wird;
(b) die Cyclisierung des Produkts der Formel (IV) mit oder ohne intermediäre Isolierung unter Rückfluß des Reaktionsgemisches in dem Medium eines Lösungsmittels, wie Methanol, oder durch Rühren bei Raumtemperatur in dem Medium eines Lösungsmittels, wie Methanol, in Gegenwart einer Base, wie Kaliumcarbonat, durchgeführt wird.

8. Variante des Verfahrens zur Herstellung der Produkte der Formel I sowie von deren Salzen, dadurch gekennzeichnet, daß man ein Produkt der Formel (II)

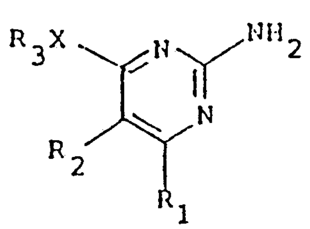

(II)

worin $R_1$, $R_2$, $R_3$ und X die angegebene Bedeutung besitzen, mit einem Produkt der Formel
$A-CH_2-CO-CO_2Q$
worin A eine nucleophile Gruppe darstellt und Q eine veresternde Gruppe ist, umsetzt, das erhaltene Produkt der Formel

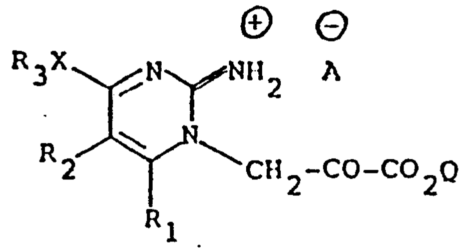

worin $R_1$, $R_2$, $R_3$, Q und X die angegebene Bedeutung besitzen und $A^-$ ein sich von A ableitendes Anion darstellt, cyclisiert, um das Produkt der Formel (IX)

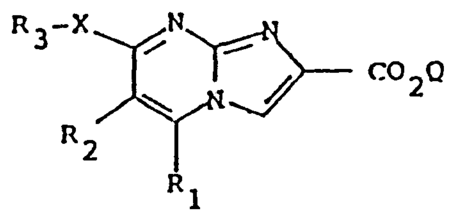

(IX)

worin $R_1$, $R_2$, $R_3$, X und Q die angegebene Bedeutung besitzen, zu erhalten, das Produkt der Formel (IX) reduziert, um das Produkt der Formel (VIII)

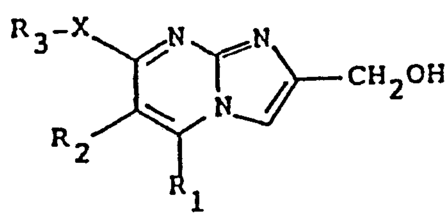

(VIII)

worin $R_1$, $R_2$, $R_3$ und X die angegebene Bedeutung besitzen, zu erhalten, das Produkt der Formel (VIII) oxidiert, um das Produkt der Formel (VI)

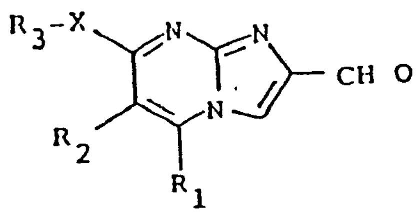

(VI)

worin $R_1$, $R_2$, $R_3$ und X die angegebene Bedeutung haben, zu erhalten, das Produkt der Formel (VI) mit einem Magnesiumderivat eines Produktes der Formel (VII)

R - K (VII)

worin R die angegebene Bedeutung besitzt und K ein Lithiumatom oder einen Rest -MgHal darstellt, worin Hal ein Chlor-, Brom- oder Jodatom bedeutet, umsetzt, um ein Produkt der Formel (V)

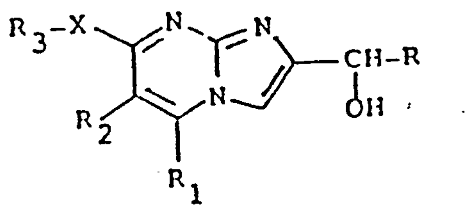 (V)

zu erhalten, worin R, $R_1$, $R_2$, $R_3$ und X die angegebene Bedeutung besitzen, danach das Produkt der Formel (V) oxidiert, um das erwartete Produkt der Formel (I) zu erhalten, das man gegebenenfalls in ein Salz überführen kann.

9. Verfahren gemäß Anspruch 8, dadurch gekennzeichnet, daß

(a) die zu dem Produkt der Formel IX führende Cyclisierung unter den gleichen Bedingungen wie derjenigen des Produkts der Formel IV, wie in Anspruch 7 angegeben, durchgeführt wird;

(b) Q einen Alkyl- oder Aralkylrest und insbesondere einen Alkylrest mit 1 bis 3 Kohlenstoffatomen, wie einen Ethylrest, darstellt;

(c) die Reduktion des Produkts der Formel IX mit Lithiumaluminiumhydrid oder auch mit Natriumborhydrid in Gegenwart von Aluminiumchlorid oder Lithiumborhydrid durchgeführt wird;

(d) die Oxidation des Produkts der Formel VIII mit Hilfe von Mangandioxid oder unter den für die Oxidation des Produkts der Formel V nachstehend angegebenen Bedingungen durchgeführt wird;

(e) die Umsetzung des Produkts der Formel VI mit dem Produkt der Formel VII unter wasserfreien Bedingungen in einem organischen Lösungsmittel, wie Tetrahydrofuran, durchgeführt wird;

(f) die Oxidation des Produkts der Formel V mit Mangandioxid, Salpetersäure, Ferrichlorid oder Chromoxid in Anwesenheit von Pyridin oder auch nach der Oppenauer-Methode oder schließlich durch Dehydrierung in Anwesenheit eines Katalysators auf Basis von Kupfer durchgeführt wird.

10. Arzneimittel, dadurch gekennzeichnet, daß sie aus den neuen Imidazo[1.2-a]pyrimidinen der Formel (I) gemäß Anspruch 1 oder aus deren pharmazeutisch verträglichen Salzen bestehen.

11. Arzneimittel, dadurch gekennzeichnet, daß sie aus den neuen Imidazo[1.2-a]pyrimidinen gemäß einem der Ansprüche 2, 3, 4 oder 5 oder aus deren pharmazeutisch verträglichen Salzen bestehen.

12. Pharmazeutische Zusammensetzungen, dadurch gekennzeichnet, daß sie als Wirkstoff zumindest eines der Arzneimittel gemäß einem der Ansprüche 10 oder 11 enthalten.

13. Als Zwischenprodukte, die für die Herstellung der Produkte der Formel I gemäß Anspruch 1 erforderlich sind, die Produkte der Formel IV gemäß Anspruch 6.

14. Als Zwischenprodukte, die für die Herstellung der Produkte der Formel (I) gemäß Anspruch 1 erforderlich sind, die Produkte der Formel

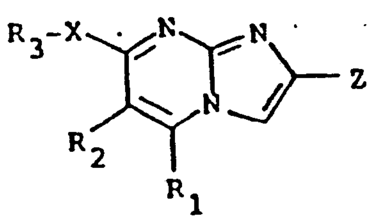

worin $R_1$, $R_2$, $R_3$ und X wie vorstehend definiert sind und Z einen Rest -CHO oder

$$\underset{OH}{\underset{|}{-CH}}$$

-R' bedeutet, worin R' ein Wasserstoffatom oder einen Rest R, wie vorstehend definiert, darstellt.

**Patentansprüche**

für den Vertragsstaat: AT

1. Verfahren zur Herstellung neuer Imidazo[1,2-a]-pyrimidine der Formel (I)

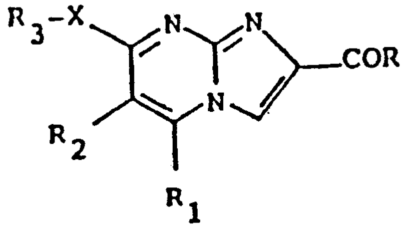

(I)

worin R einen linearen Alkylrest mit 1 bis 8 Kohlenstoffatomen, einen linearen Alkenylrest mit 2 bis 8 Kohlenstoffatomen, einen Cycloalkylrest mit 3 bis 7 Kohlenstoffatomen, einen (Cycloalkyl)-alkylrest mit 4 bis 12 Kohlenstoffatomen, einen Arylrest mit 6 bis 12 Kohlenstoffatomen oder einen Thienyl- oder Pyridylremt bedeutet, wobei ein jeder der vorstehenden Reste durch ein oder mehrere Halogenatome, einen oder mehrere Alkyl-, Alkylthio- oder Alkoxyreste mit 1 bis 5 Kohlenstoffatomen, einen oder mehrere Amino-, Alkylamino- oder Dialkylaminoreste, worin die Alkylreste 1 bis 5 Kohlenstoffatome enthalten, oder durch einen oder mehrere Trifluormethylreste substituiert sein kann,

$R_1$ und $R_2$, die gleich oder verschieden sind, ein Wasserstoffatom, einen linearen Alkylrest mit 1 bis 8 Kohlenstoffatomen, einen linearen Alkenylrest mit 2 bis 5 Kohlenstoffatomen, einen Cycloalkylrest mit 3 bis 7 Kohlenstoffatomen, einen (Cycloalkyl)-alkylrest mit 4 bis 12 Kohlenstoffatomen, einen Benzylrest oder einen Phenethylrest bedeuten oder $R_1$ und $R_2$ gemeinsam einen linearen Alkylenrest mit 3 bis 5 Kohlenstoffatomen bilden, wobei ein jeder der vorstehenden Reste durch ein oder mehrere Halogenatome, einen oder mehrere Alkyl-, Alkoxy- oder Alkylthioreste mit 1 bis 5 Kohlenstoffatomen, durch einen oder mehrere Amino-, Alkylamino- oder Dialkylaminoreste, worin die Alkylreste 1 bis 5 Kohlenstoffatome enthalten, oder durch einen oder mehrere Trifluormethylreste substituiert sein kann, X ein Sauerstoff- oder Schwefelatom bedeutet und $R_3$ einen Alkylrest mit 1 bis 5 Kohlenstoffatomen bedeutet,

sowie von deren Additionssalzen mit Mineral- oder organischen Säuren, dadurch gekennzeichnet, daß man entweder ein Produkt der Formel (II)

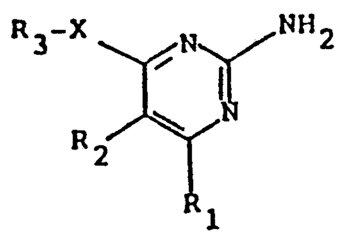

(II)

worin $R_1$, $R_2$, $R_3$ und X die angegebene Bedeutung besitzen, mit einem Produkt der Formel (III)

$Y\text{-}CH_2\text{-}CO\text{-}CO\text{-}R$ (III)

umsetzt, worin R die angegebene Bedeutung besitzt und Y eine Gruppe darstellt, die durch Eliminierung ein Anion $Y^-$ bilden kann, um ein Produkt der Formel (IV)

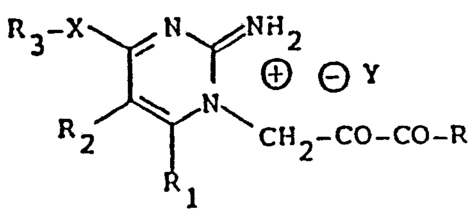

(IV)

zu erhalten, worin R, $R_1$, $R_2$, $R_{3>1}$, X und Y die angegebene Bedeutung besitzen, und danach das erhaltene Produkt der Formel (IV) cyclisiert, um ein Produkt der Formel (I) zu erhalten, das man gegebenenfalls in ein Salz überführen kann;

oder ein Produkt der Formel (II)

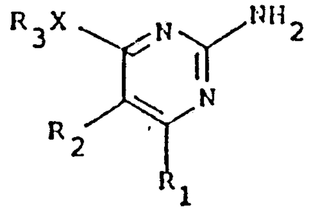

(II)

worin $R_1$, $R_2$, $R_3$ und X die angegebene Bedeutung besitzen, mit einem Produkt der Formel

$A\text{-}CH_2\text{-}CO\text{-}CO_2Q$

worin A eine nucleophile Gruppe darstellt und Q eine veresternde Gruppe ist, umsetzt, das erhaltene Produkt der Formel

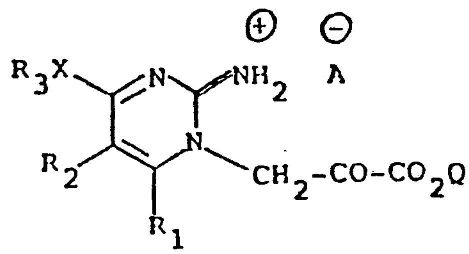

worin $R_1$, $R_2$, $R_3$, Q und X die angegebene Bedeutung besitzen und $A^-$ ein sich von A ableitendes Anion darstellt, cyclisiert, um das Produkt der Formel (IX)

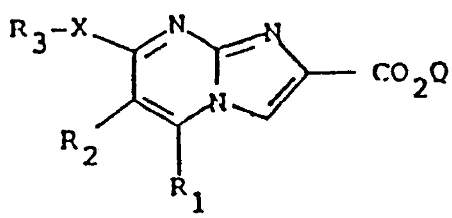

(IX)

worin $R_1$, $R_2$, $R_3$, X und Q die angegebene Bedeutung besitzen, zu erhalten, das Produkt der Formel (IX) reduziert, um das Produkt der Formel (VIII)

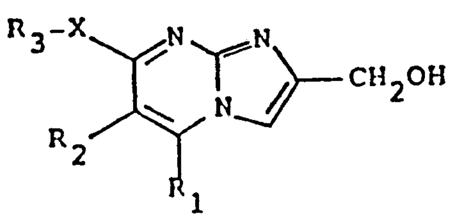

(VIII)

worin $R_1$, $R_2$, $R_3$ und X die angegebene Bedeutung besitzen, zu erhalten, das Produkt der Formel (VIII) oxidiert, um das Produkt der Formel (VI)

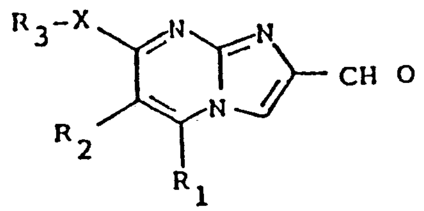

(VI)

worin $R_1$, $R_2$, $R_3$ und X die angegebene Bedeutung haben, zu erhalten, das Produkt der Formel (VI) mit einem Magnesiumderivat eines Produktes der Formel (VII)

R - K (VII)

worin R die angegebene Bedeutung besitzt und K ein Lithiumatom oder einen Rest -MgHal darstellt, worin Hal ein Chlor-, Brom- oder jodatom bedeutet, umsetzt, um ein Produkt der Formel (V)

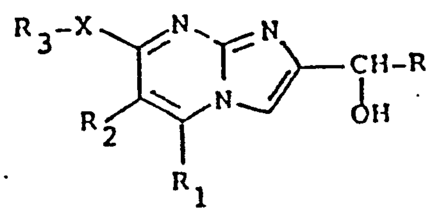

(V)

zu erhalten, worin R, $R_1$, $R_2$, $R_3$ und X die angegebene Bedeutung besitzen, danach das Produkt der Formel (V) oxidiert, um das erwartete Produkt der Formel (I) zu erhalten, das man gegebenenfalls in ein Salz überführen kann.

2. Verfahren gemäß Anspruch 1 zur Herstellung der Produkte der Formel I gemäß Anspruch 1 sowie von deren Salzen, dadurch gekennzeichnet, daß man ein Produkt der Formel (II)

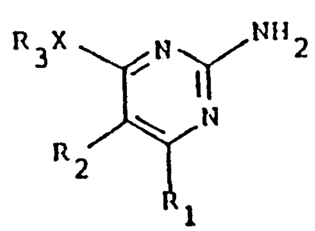

(II)

worin $R_1$, $R_2$, $R_3$ und X die angegebene Bedeutung besitzen, mit einem Produkt der Formel (III)

$Y-CH_2-CO-CO-R$ (III)

umsetzt, worin R die angegebene Bedeutung besitzt und Y eine Gruppe ist, die durch Eliminierung ein Anion $Y^-$ bilden kann, um ein Produkt der Formel (IV)

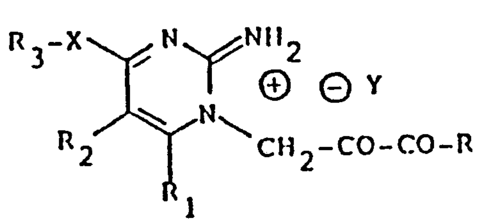

(IV)

zu erhalten, worin R, $R_1$, $R_2$, $R_3$, X und Y die angegebene Bedeutung besitzen, und danach das erhaltene Produkt der Formel (IV) cyclisiert, um ein Produkt der Formel (I) zu erhalten, das man gegebenenfalls in ein Salz überführen kann.

3. Verfahren gemäß Anspruch 1, dadurch gekennzeichnet, daß

(a) die Umsetzung des Produkts der Formel (II) mit dem Produkt der Formel (III) in dem Medium eines

organischen Lösungsmittels, wie Ethylether, Tetrahydrofuran oder Dimethoxyethan, durchgeführt wird;

(b) die Cyclisierung des Produkts der Formel (IV) mit oder ohne intermediäre Isolierung unter Rückfluß des Reaktionsgemisches in dem Medium eines Lösungsmittels, wie Methanol, oder durch Rühren bei Raumtemperatur in dem Medium eines Lösungsmittels, wie Methanol in Gegenwart einer Base, wie Kaliumcarbonat, durchgeführt wird.

4. Verfahren gemäß Anspruch 1 zur Herstellung der Produkte der Formel (I) gemäß Anspruch 1 sowie von deren Salzen, dadurch gekennzeichnet, daß man ein Produkt der Formel (II)

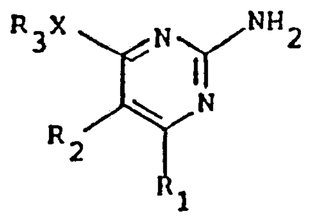

(II)

worin $R_1$, $R_2$, $R_3$ und X die angegebene Bedeutung besitzen, mit einem Produkt der Formel

$A-CH_2-CO-CO_2Q$

worin A eine nucleophile Gruppe darstellt und Q eine veresternde Gruppe ist, umsetzt, das erhaltene Produkt der Formel

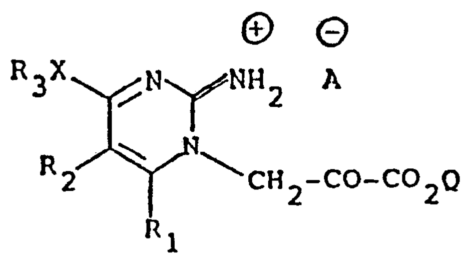

worin $R_1$, $R_2$, $R_3$, Q und X die angegebene Bedeutung besitzen und $A^-$ ein sich von A ableitendes Anion darstellt, cyclisiert, um das Produkt der Formel (IX)

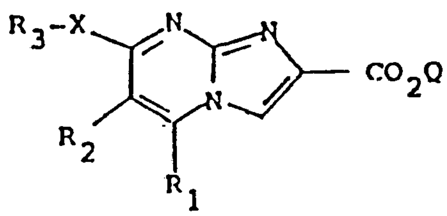

(IX)

worin $R_1$, $R_2$, $R_3$, X und Q die angegebene Bedeutung besitzen, zu erhalten, das Produkt der Formel (IX) reduziert, um das Produkt der Formel (VIII)

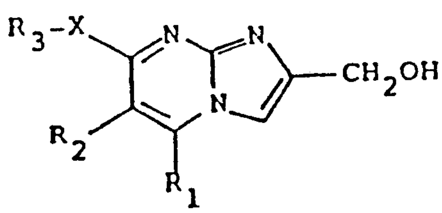

(VIII)

worin $R_1$, $R_2$, $R_3$ und X die angegebene Bedeutung besitzen, zu erhalten, das Produkt der Formel (VIII) oxidiert, um das Produkt der Formel (VI)

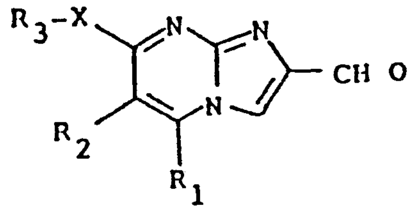

(VI)

worin $R_1$, $R_2$, $R_3$ und X die angegebene Bedeutung haben, zu erhalten, das Produkt der Formel (VI) mit einem Magnesiumderivat eines Produktes der Formel (VII)

R - K (VII)

worin R die angegebene Bedeutung besitzt und K ein Lithiumatom oder einen Rest -MgHal darstellt, worin Hal ein Chlor-, Brom- oder jodatom bedeutet, umsetzt, um ein Produkt der Formel (V)

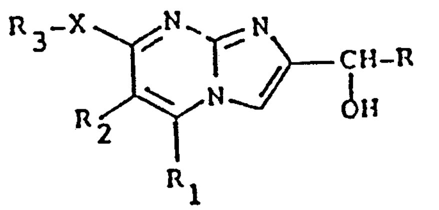

(V)

zu erhalten, worin R, $R_1$, $R_2$, $R_3$ und X die angegebene Bedeutung besitzen, danach das Produkt der Formel (V) oxidiert, um das erwartete Produkt der Formel (I) zu erhalten, das man gegebenenfalls in ein Salz überführen kann.

5. Verfahren gemäß Anspruch 4, dadurch gekennzeichnet, daß

(a) die zu dem Produkt der Formel (IX) führende Cyclisierung unter den gleichen Bedingungen durchgeführt wird wie diejenige des Produkts der Formel (IV), wie in Anspruch 3 angegeben;

(b) Q einen Alkyl- oder Aralkylrest und insbesondere einen Alkylrest mit 1 bis 3 Kohlenstoffatomen, wie einen Ethylrest, bedeutet;

(c) die Reduktion des Produkts der Formel (IX) mit Lithiumaluminiumhydrid oder auch mit Natriumborhydrid in Anwesenheit von Aluminiumchlorid oder Lithiumborhydrid durchgeführt wird;

(d) die Oxidation des Produkts der Formel (VIII) mit Hilfe von Mangandioxid oder unter den nachstehend für die Oxidation des Produkts der Formel (V) angegebenen Bedingungen durchgeführt wird;

(e) die Umsetzung des Produkts der Formel (VI) mit dem Produkt der Formel (VII) unter wasserfreien Bedingungen in einem organischen Lösungsmittel, wie Tetrahydrofuran, durchgeführt wird;

(f) die Oxidation des Produkts der Formel (V) mit Mangandioxid, Salpetersäure, Ferrichlorid oder Chromoxid in Anwesenheit von Pyridin oder auch nach der Oppenauer-Methode oder schließlich durch Dehydrierung in Gegenwart eines Katalysators auf Basis von Kupfer durchgeführt wird;

6. Verfahren gemäß einem der Ansprüche 1 bis 5, dadurch gekennzeichnet, daß man von einem Produkt der Formel (III) oder (VII) ausgeht, worin R einen Phenylrest bedeutet.

7. Verfahren gemäß einem der Ansprüche 1 bis 6, dadurch gekennzeichnet, daß man von einem Produkt der Formel (II) ausgeht, worin $R_1$ und $R_2$, die gleich oder verschieden sind, ein Wasserstoffatom, einen Methyl-, Ethyl-, Propyl-, Isopropyl-, Hexyl-, Allyl- oder Benzylrest bedeuten oder $R_1$ und $R_2$ gemeinsam einen 1,3-Propylen-, 1,4-Butylen-, 1,5-Pentylenrest oder einen Rest

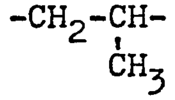

$CH_2$-$CH_2$- bilden und $R_3$ einen Methyl- oder Ethylrest bedeutet, wobei R und X die vorstehend angegebene Bedeutung besitzen.

8. Verfahren gemäß einem der Ansprüche 1 bis 7, dadurch gekennzeichnet, daß man von Produkten ausgeht, die derart ausgewählt sind, daß das [6-Ethyl-5-methyl-7-(methylthio)-imidazo[1.2-a]pyrimidin-2-yl]-phenylmethanon und dessen Salze hergestellt werden.

9. Verfahren gemäß einem der Ansprüche 1 bis 7, dadurch gekennzeichnet, daß man von Produkten ausgeht, die derart ausgewählt sind, daß man eines der Produkte der Formel I mit den folgenden Bezeichnungen herstellt:

(5-Methoxy-6,7,8,9-tetrahydroimidazo[1.2-a]-chinazolin-2-yl)-phenylmethanon,

[5-(Methylthio)-cyclopenta-[e]-imidazo[1.2-a]-pyrimidin-2-yl]-phenylmethanon,

[7-Methoxy-5-methyl-6-(1-methylethyl)-imidazo-[1.2-a]pyrimidin-2-yl]-phenylmethanon und

(6-Ethyl-7-methoxy-5-methylimidazo[1.2-a]pyrimi-din-2-yl)-phenylmethanon sowie deren Salze.

## Claims

for the contracting states: BE, CH, DE, FR, IT, LI, LU, NL, SE

1) New imidazo[1,2-a]pyrimidines answering to the formula (I):

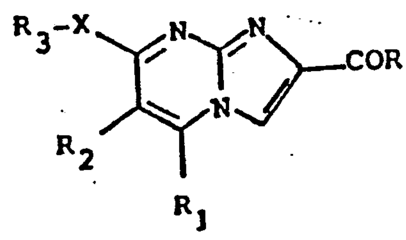

(I)

in which R represents a linear alkyl radical containing from 1 to 8 carbon atoms, a linear alkenyl radical containing from 2 to 8 carbon atoms, a cycloalkyl radical containing from 3 to 7 carbon atoms, a (cycloalkyl)alkyl radical containing from 4 to 12 carbon atoms, an aryl radical containing from 6 to 12 carbon atoms or a thienyl or pyridyl radical, each of the above radicals being able to be substituted by one or more halogen atoms, one or more alkyl, alkylthio or alkoxy radicals containing from 1 to 5 carbon atoms, one or more amino, alkylamino or dialkylamino radicals, in which the alkyl radicals contain from 1 to 5 carbon atoms, or by one or more trifluoromehyl radicals;

$R_1$ and $R_2$, identical or different, represent a hydrogen atom, a linear alkyl radical containing from 1 to 8 carbon atoms, a linear alkenyl radical containing from 2 to 5 carbon atoms, a cycloalkyl radical containing from 3 to 7 carbon atoms, a (cycloalkyl)alkyl radical containing from 4 to 12 carbon atoms, a benzyl radical or a phenethyl radical, or $R_1$ and $R_2$ together form a linear alkylene radical containing from 3 to 5 carbon atoms, each of the above radicals being able to be substituted by one or more halogen atoms, one or more alkyl, alkoxy or alkylthio radicals containing from 1 to 5 carbon atoms, by one or more amino, alkylamino or dialkylamino radicals in which the alkyl radicals contain from 1 to 5 carbon atoms, or by one or more trifluoromethyl radicals, X represents an oxygen or sulphur atom and $R_3$ represents an alkyl radical containing from 1 to 5 carbon atoms, as well as their salts of addition with mineral or organic acids.

2) New imidazo[1,2-a]pyrimidines as defined in claim 1, and their salts, characterized in that in the said formula (I), R represents a phenyl radical, $R_1$, $R_2$, $R_3$ and X have the significance already indicated.

3) New imidazo[1,2-a]pyrimidines, as defined in claim 1 or 2, and their salts, characterized in that in the said formula (I), $R_1$ and $R_2$, identical or different, represent a hydrogen atom, a methyl, ethyl, propyl, isopropyl, hexyl, allyl or benzyl radical or $R_1$ and $R_2$ together form a 1, 3-propylene, 1,4-butylene or 1,5-pentylene radical, or a

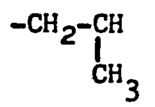

$-CH_2-CH_2-$ radical, $R_3$ represents a methyl or ethyl radical, R and X having the significance already indicated.

4) [6-ethyl-5-methyl-7- (methylthio) imidazo[ 1, 2-a]pyrimidin-2-yl]-phenylmethanone and its salts.

5) Any one of the products with the formula (I) the names of which follow:

- (5-methoxy-6,7,8,9-tetrahydroimidazo[ 1,2-a]quinazolin-2-yl)-phenylme thanone,
- [5- (methylthio) -cyclopenta [e ]imidazo(I, 2-a]pyrimidin-2-yl] -phenylmethanone,
- [7-methoxy-5-methyl-6- (1 -methylethyl) -imidazo[ 1,2-a] pyrimidin-2-yl]-phenylmethanone and
- (6-ethyl-7-methoxy-5-methyl-imidazo[ 1,2-a]pyrimidin-2-yl)-phenylmethanone as well as their salts.

6) Preparation process for new imidazo[1,2-a]pyrimidines as defined by the formula (I) of claim 1, as well as their salts, characterized in that a product with the formula (II):

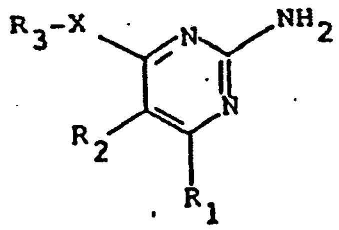

(II)

in which $R_1$, $R_2$, $R_3$ and X have the significance already indicated, is made to react with a product with the formula (III):

Y-$CH_2$-CO-CO-R (III)

in which R has the significance already indicated and Y is a group able to form by elimination, an anion $Y^{\ominus}$ so as to obtain a product with the formula (IV):

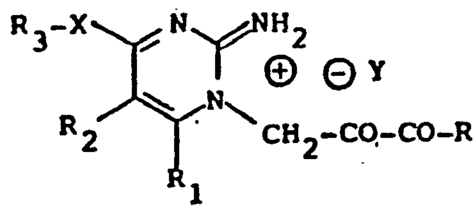

(IV)

in which R, R1, $R_2$, $R_3$, X and Y have the significance already indicated, then the said product with the formula (IV) obtained is cyclised so as to obtain a product with the formula (I) which can be salified if the case arises.

7) Preparation process according to claim 6, characterized in that:

a) the reaction of the product with the formula (II) with the product with the formula (III) is carried out in an organic solvent such as ethyl ether, tetrahydrofuran or dimethoxyethane; b) the cyclisation of the product with the formula (IV) with or without intermediate isolation is carried out at reflux of the reactional mixture in a solvent such as methanol or by agitation at ambient temperature, in a solvent such as methanol, in the presence of a base such as potassium carbonate.

8) Variation of the preparation process for products with the formula (I), as well as their salts, characterized in that a product with the formula (II):

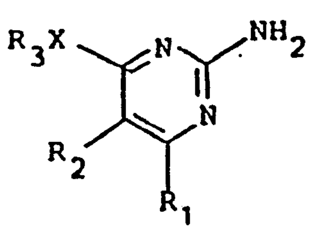

(II)

in which R1, $R_2$, $R_3$ and X have the significance already indicated, is made to react with a product with the formula:

A-$CH_2$-CO-$CO_2$Q

in which A represents a nucleophile group and Q represents an esterifying group, the product obtained with the formula:

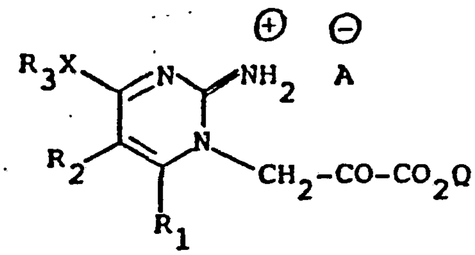

in which $R_1$, $R_2$, $R_3$, Q and X have the significance already indicated and $A^{\ominus}$ represents an anion derivative of A, is cyclised so as to obtain the product with the formula (IX):

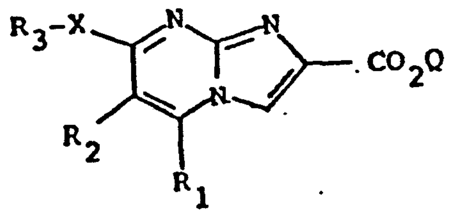
(IX)

in which $R_1$, $R_2$, $R_3$, X and Q have the significance already indicated, the product with the formula (IX) is reduced so as to obtain the product with the formula (VIII):

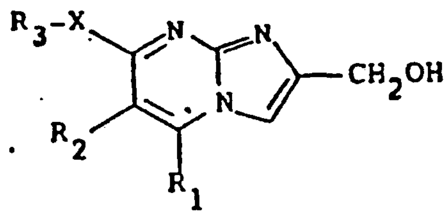
(VIII)

in which $R_1$, $R_2$, $R_3$ and X have the significance already indicated, the product with the formula (VIII) is oxidized so as to obtain the product with the formula (VI):

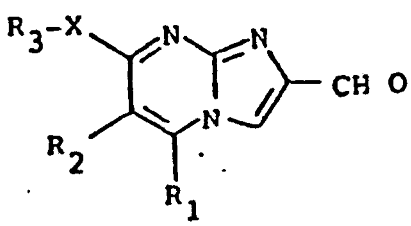
(VI)

in which $R_1$, $R_2$, $R_3$ and X have the significance already indicated, the product with the formula (VI) is made to react with a magnesium derivative of a product with the formula (VII):

R-K (VII)

in which R has the significance already indicated and K represents a lithium atom or a -MgHal residue, in which Hal represents a chlorine, bromine or iodine atom, so as to obtain a product with the formula (V):

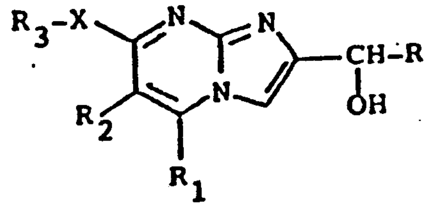 (V)

in which R, $R_1$, $R_2$, $R_3$ and X have the significance already indicated, then the product with the formula (V) is oxidized so as to obtain the expected product with the formula (I) which can be salified if the case arises.

9) Preparation process according to Claim 8, characterized in that:

a) the cyclisation leading to the product with the formula (IX) is carried out in the same conditions as those of the product with the formula (IV) indicated in Claim 7;

b) Q is an alkyl or aralkyl radical and more particularly an alkyl radical containing from 1 to 3 carbon atoms such as an ethyl radical;

c) the reduction of the product with the formula (IX) is carried out by lithium-aluminium hydride or also by sodium borohydride in the presence of aluminium chloride or lithium borohydride.

d) the oxidation of the product with the formula (VIII) is carried out by manganese dioxide or in conditions indicated below for the oxidation of the product with the formula (V);

e) the reaction of the product with the formula (VI) with the product with the formula (VII) is carried out in anhydrous conditions in an organic solvent such as tetrahydrofuran;

f) the oxidation of the product with the formula (V) is carried out with manganese dioxide, nitric acid, ferric chloride or chrome oxide in the presence of pyridine, or also by the Oppenauer method or finally by dehydrogenation in the presence of a copper-based catalyst.

10) Medicaments characterized in that they are composed of the new imidazo[1,2-a]pyrimidines as defined by the formula (I) of claim 1, as well as by their pharmaceutically acceptable salts.

11) Medicaments characterized in that they are composed of new imidazo[l,2-a]pyrimidines as defined in any one of the claims 2, 3, 4 or 5, as well as by their pharmaceutically acceptable salts.

12) Pharmaceutical compositions, characterized in that they contain as active principle, at least one of the medicaments as defined in any one of the claims 10 or 1 1.

13) As intermediates necessary for the preparation of products with the formula (I), as defined in claim 1, products with the formula (IV), as defined in claim 6.

14) As intermediates necessary for the preparation of products with the formula (I), as defined in claim 1, products with theformula:

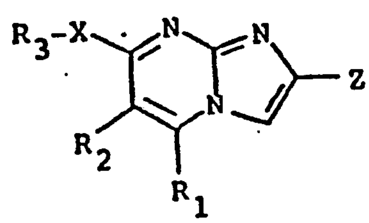

in which $R_1$, $R_2$, $R_3$ and X are defined as previously and Z represents a radical

-CHO or 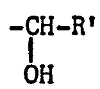

in which R'represents a hydrogen atom or a radical R as defined previously.

**Claims**

for the contracting state: AT

1) Preparation process for new imidazo[ 1,2-a]pyrimidines answering to the formula (I):

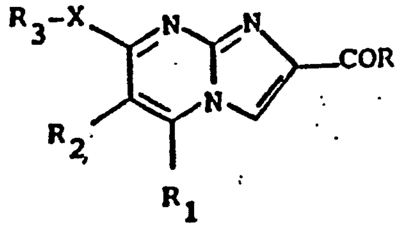

(I)

in which R represents a linear alkyl radical containing from 1 to 8 carbon atoms, a linear alkenyl radical containing from 2 to 8 carbon atoms, a cycloalkyl radical containing from 3 to 7 carbon atoms, a (cycloalkyl)alkyl radical containing from 4 to 12 carbon atoms, an aryl radical containing from 6 to 12 carbon atoms or a thienyl or pyridyl radical, each of the above radicals being able to be substituted by one or more halogen atoms, one or more alkyl, alkylthio or alkoxy radicals containing from 1 to 5 carbon atoms, one or more amino, alkylamino or dialkylamino radicals, in which the alkyl radicals contain from 1 to 5 carbon atoms, or by one or more trifluoromethyl radicals;

$R_1$ and $R_2$, identical or different, represent a hydrogen atom, a linear alkyl radical containing from 1 to 8 carbon atoms, a linear alkenyl radical containing from 2 to 5 carbon atoms, a cycloalkyl radical containing from 3 to 7 carbon atoms, a (cycloalkyl)alkyl radical containing from 4 to 12 carbon atoms, a benzyl radical or a phenethyl radical, or $R_1$ and $R_2$ together form a linear alkylene radical containing from 3 to 5 carbon atoms, each of the above radicals being able to be substituted by one or more halogen atoms, one or more alkyl, alkoxy or alkylthio radicals containing from 1 to 5 carbon atoms, by one or more amino, alkylamino or dialkylamino radicals in which the alkyl radicals contain from 1 to 5 carbon atoms, or by one or more trifluoromethyl radicals, X represents an oxygen or sulphur atom and $R_3$ represents an alkyl radical containing from 1 to 5 carbon atoms, as well as their salts of addition with mineral or organic acids characterized in that

- <u>either</u> a product with the formula (II)

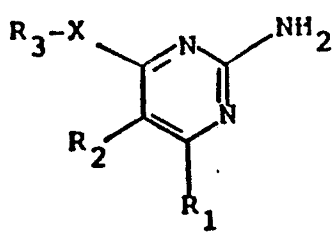

(II)

in which $R_1$, $R_2$, $R_3$ and X have the significance already indicated, is made to react with a product with the formula (III):

$Y\text{-}CH_2\text{-}CO\text{-}CO\text{-}R$ (III)

in which R has the significance already indicated and Y is a group able to form by elimination, an anion $Y^{\ominus}$ so as to obtain a product with the formula (IV):

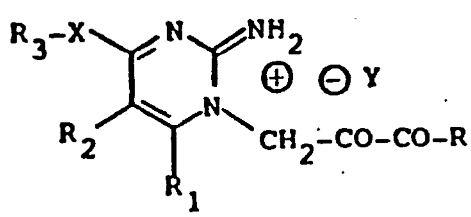

(IV)

in which R, $R_1$, $R_2$, $R_3$, X and Y have the significance already indicated, then the said product with the formula (IV) obtained is cyclised so as to obtain a product with the formula (I) which can be salified if the case arises,

- <u>or</u> a product with the formula (II)

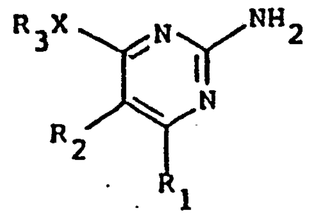

(II)

in which Rl, $R_2$, $R_3$ and X have the significance already indicated, is made to react with a product with the formula:

$A-CH_2-CO-CO_2Q$

in which A represents a nucleophile group and Q represents an esterifying group, the product obtained with the formula:

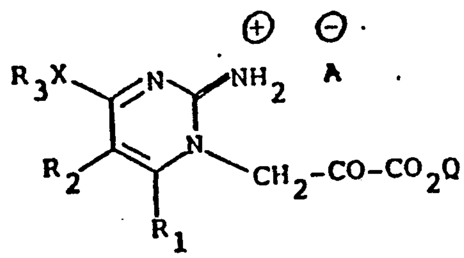

in which $R_1$, $R_2$, $R_3$, Q and X have the significance already indicated and $A^{\ominus}$ represents an anion derivative of A, is cyclised so as to obtain the product with the formula (IX):

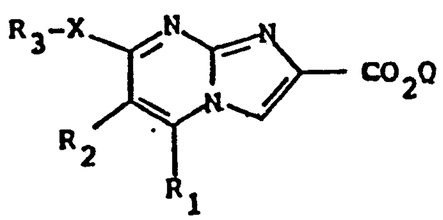

(IX)

in which $R_1$, $R_2$, $R_3$, X and Q have the significance already indicated, the product with the formula (IX) is reduced so as to obtain the product with the formula (VIII):

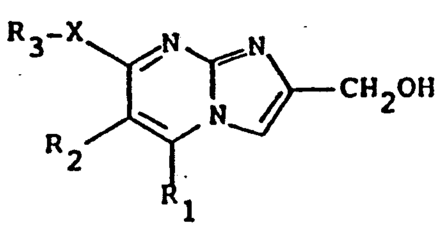

(VIII)

in which $R_1$, $R_2$, $R_3$ and X have the significance already indicated, the product with the formula (VIII) is oxidized so as to obtain the product with the formula (VI):

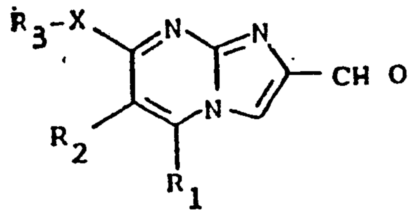

(VI)

in which $R_1$, $R_2$, $R_3$ and X have the significance already indicated, the product with the formula (VI) is made to react with a magnesium derivative of a product with the formula (VII):

R-K (VII)

in which R has the significance already indicated and K represents a lithium atom or a -MgHal residue, in which Hal represents a chlorine, bromine or iodine atom, so as to obtain a product with the formula (V):

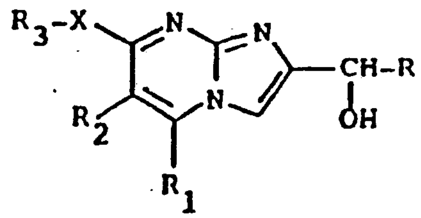

(V)

in which R $R_1$ $R_2$, $R_3$ and X have the significance already indicated, then the product with the formula (V) is oxidized so as to obtain the expected product with the formula (I) which can be salified if the case arises.

2) Process according to claim 1, for the preparation of products with the formula (I), as defined in claim 1, as well as their salts, characterized in that a product with the formula (II):

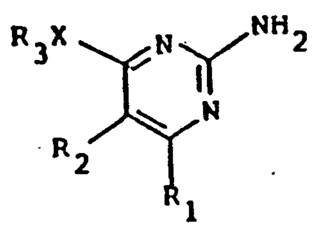

(II)

in which $R_1$, $R_2$, $R_3$ and X have the significance already indicated, is made to react with a product with the formula (III):

$Y-CH_2-CO-CO-R$ (III)

in which R has the significance already indicated and Y is a group able to form by elimination, an anion $Y^{\ominus}$ so as to obtain a product with the formula (IV):

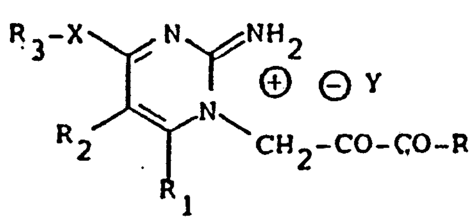

(IV)

in which R, $R_1$, $R_2$, $R_3$, X and Y have the significance already indicated, then the said product with the formula (IV) obtained is cyclised so as to obtain a product with the formula (I) which can be salified if the case arises.

3) Preparation process according to claim 1, characterized in that:

a) the reaction of the product with the formula (II) with the product with the formula (III) is carried out in an organic solvent such as ethyl ether, tetrahydrofuran or dimethoxyethane;

b) the cyclisation of the product with the formula (IV) with or without intermediate isolation is carried out at reflux of the reactional mixture in a solvent such as methanol or by agitation at ambient temperature, in a solvent such as methanol, in the presence of a base such as potassium carbonate.

4) Process acording to claim 1, for the preparation of products with the formula (I), as defined in claim 1, as well as their salts, characterized in that:

a product with the formula (II):

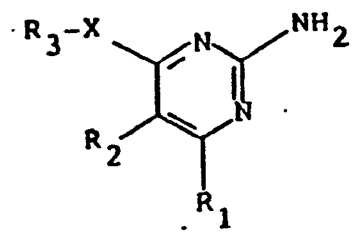

(II)

in which $R_1$, $R_2$, $R_3$ and X have the significance already indicated, is made to react with a product with the formula:

$A-CH_2-CO-CO_2Q$

in which A represents a nucleophile group and Q represents an esterifying group, the product obtained with the formula:

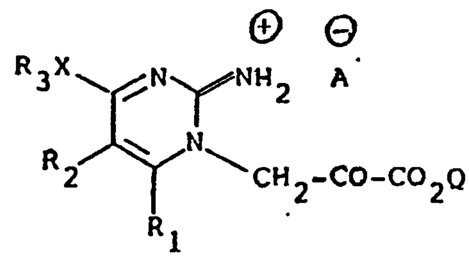

in which $R_1$, $R_2$, $R_3$, Q and X have the significance already indicated and $A^{\ominus}$ represents an anion derivative of A, is cyclised so as to obtain the product with the formula (IX):

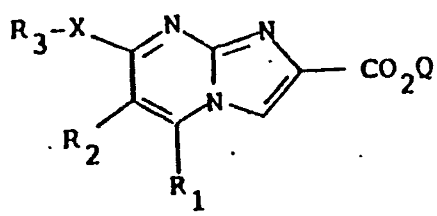

(IX)

in which R1, $R_2$, $R_3$ X and Q have the significance already indicated, the product with the formula (IX) is reduced so as to obtain the product with the formula (VIII):

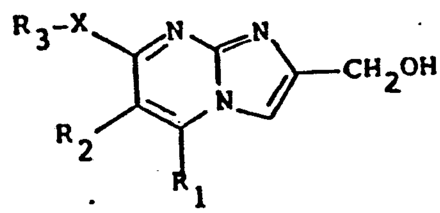

(VIII)

in which $R_1$, $R_2$, $R_3$ and X have the significance already indicated, the product with the formula (VIII) is oxidized so as to obtain the product with the formula (VI):

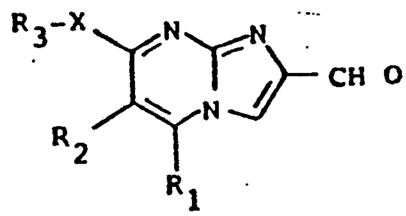

(VI)

in which $R_1$, $R_2$, $R_3$ and Y have the significance already indicated, the product with the formula (VI) is made to react with a magnesium derivative of a product with the formula (VII):

R-K (VII)

in which R has the significance already indicated and K represents a lithium atom or a -MgHal residue, in which Hal represents a chlorine, bromine or iodine atom, so as to obtain a product with the formula (V):

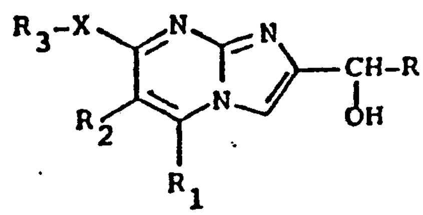

(V)

in which R, $R_1$, $R_2$, $R_3$ and X have the significance already indicated, then the product with the formula (V) is oxidized so as to obtain the expected product with the formula (I) which can be salified if the case arises.

5) Preparation process according to Claim 4, characterized in that:

a) the cyclisation leading to the product with the formula (IX) is carried out in the same conditions as those of the product with the formula (IV) indicated in Claim 3;

b) Q is an alkyl or aralkyl radical and more particularly an alkyl radical containing from 1 to 3 carbon atoms such as an ethyl radical;

c) the reduction of the product with the formula (IX) is carried out by lithium-aluminium hydride or also by sodium borohydride in the presence of aluminium chloride or lithium borohydride.

d) the oxidation of the product with the formula (VIII) is carried out by manganese dioxide or in conditions indicated below for the oxidation of the product with the formula (V);

e) the reaction of the product with the formula (VI) with the product with the formula (VII) is carried out in anhydrous conditions in an organic solvent such as tetrahydrofuran;

f) the oxidation of the product with the formula (V) is carried out with manganese dioxide, nitric acid, ferric chloride or chrome oxide in the presence of pyridine, or also by the Oppenauer method or finally by dehydrogenation in the presence of a copper-based catalyst.

6) Process according to any one of the claims 1 to 5, characterized in that at the start, a product with the formula (III) or (VII) is used in which R represents a phenyl radical.

7) Process according to any one of the claims 1 to 6, characterized in that at the start a product with the formula (II) is used in which $R_1$ and $R_2$, identical or different, represent a hydrogen atom, a methyl, ethyl, propyl, isopropyl, hexyl, allyl or benzyl radical or $R_1$ and $R_2$ together form a 1, 3-propylene, 1,4-butylene or 1,5-pentylene radical, or a

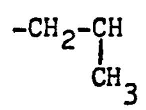

$-CH_2-CH_2$-radical,

$R_3$ represents a methyl or ethyl radical, R and X having the significance already indicated.

8) Process according to any one of the claims 1 to 7, characterized in that at the start products are used which are chosen in such a way that [6-ethyl-5-methyl-7-(methylthio)imidazo-(1,2-a]pyrimidin-2-yl]-phenylmethanone and its salts are prepared.

9) Process according to any one of the claims 1 to 7, characterized in that at the start products are used which are chosen in such a way that any one of the products with the formula (I) are prepared the names of which follow:

- (5-methoxy-6, 7, 8,9-tetrahydroimidazo[ 1,2-a]quinazolin-2-yl)-phenylmethanone,
- [5- (methylthio) -cyclopenta[e]imidazo[ 1, 2-a]pyrimidin-2-yl]-phenylmethanone,

- [7-methoxy-5-methyl-6-(1-methylethyl) -imidazo[ 1,2-a]pyrimidin-2-yl]-phenylmethanone and
- (6-ethyl-7-methoxy-5-methyl-imidazo[ 1, 2-a] pyrimidin-2-yl)-phenylmethanone as well as their salts.